# EUROPEAN PATENT SPECIFICATION

(11) **EP 2 196 462 B1**
(45) Date of publication and mention of the grant of the patent: **28.09.2011**
(21) Application number: 09178830.7
(22) Date of filing: 11.12.2009
(51) Int. Cl.: C07D 307/93, C07D 493/18, C07D 495/18, C08F 24/00, G03F 7/039, C07D 407/12, C07D 493/08

(54) **Polymerizable compound, lactone-containing compound, method for manufacturing lactone-containing compound and polymer compound obtained by polymerizing the polymerizable compound**
Polymerisierbare Verbindung, lactonhaltige Verbindung, Verfahren zur Herstellung der lactonhaltigen Verbindung und durch Polymerisierung der polymerisierbaren Verbindung erhaltene Polymerverbindung
Composé polymérisable, composé contenant une lactone, procédé pour la fabrication du composé contenant une lactone et composé polymère obtenu par la polymérisation du composé polymérisable

(30) Priority: 12.12.2008 JP 2008317751; 12.12.2008 JP 2008317752; 12.12.2008 JP 2008317754; 06.03.2009 JP 2009054291; 03.04.2009 JP 2009091616; 20.05.2009 JP 2009122470; 29.05.2009 JP 2009131275; 15.07.2009 JP 2009167004; 30.10.2009 JP 2009251478
(43) Date of publication of application: 16.06.2010
(73) Proprietor: Fujifilm Corporation, Minato-ku Tokyo (JP)
(72) Inventor: Iizuka, Yusuke, Shizuoka (JP); Iwato, Kaoru, Shizuoka (JP); Saegusa, Hiroshi, Shizuoka (JP); Hirano, Shuji, Shizuoka (JP)
(74) Representative: HOFFMANN EITLE

(56) References cited:
- JP-A- 2007 182 488
- JP-A- 2008 231 059

## Description

### BACKGROUND OF THE INVENTION

### 1. Field of the Invention

The present invention relates to a polymer compound which is used for resist compositions to be used in a manufacturing process of a semiconductor such as IC and manufacture of a circuit substrate of a liquid crystal, a thermal head and the like and further in a lithography process of other photofabrication and to a polymerizable compound which is used for synthesis of the polymer compound, In particular, the invention relates to a polymerizable compound which is useful as a raw material of a polymer compound to be used for a resist composition suitable for exposure by a liquid immersion type projection exposure system using far ultraviolet rays having a wavelength of not more than 300 nm as a light source and to a corresponding polymer compound.

### 2. Description of the Related Art

A resist for ArF excimer laser (193 nm) using a chemical amplification mechanism is being the main current at present. However, in the case where such a resist is subjected to liquid immersion exposure, there is involved a problem of pattern collapse that a formed line pattern collapses, resulting in a defect at the time of device manufacture, and it is still have problem in line edge roughness that a pattern side wall becomes rough.

Also, when a chemically amplified resist is applied to liquid immersion exposure, a resist layer comes into contact with an immersion liquid at the time of exposure, and therefore, it is pointed out that the resist layer is denatured, or a component which adversely affects the immersion liquid bleeds out. WO 04/068242 discloses an example in which a resist performance changes by immersing a resist for ArF exposure in water before and after exposure and points out that this matter is of a problem in the liquid immersion exposure. JP-A-2006-309245 discloses an example in which bleeding is suppressed by the addition of a resin containing silicon or fluorine.

Also, in a liquid immersion exposure process, in the case of exposure using a scanning type liquid immersion exposure machine, an exposure speed is lowered unless an immersion liquid also moves following the movement of a lens, and therefore, there is a concern that the productivity is affected, In the case where the immersion liquid is water, it is desirable that a resist film which is hydrophobic is good in water follow-up properties. On the other hand, in order to make deterioration of a resist pattern profile low and to suppress the generation of a scum, an affinity of a lactone group or the like with a developing solution is required, and it is necessary to make these performances compatible with each other. For example, JP-A-2007-182488 discloses a polymer compound composed of a repeating unit having a fluorinated alkyl ester introduced into norbornene lactone, but the affinity with a developing solution is low. JP-A-2008-23 1059 discloses a polymer compound containing a repeating unit having a lactone structure in which an electron withdrawing group is substituted thereon.

### SUMMARY OF THE INVENTION

An aspect of the invention is to provide a novel polymerizable compound, a lactone-containing compound, a method for manufacturing the lactone-containing compound and a polymer compound obtained by polymerizing the polymerizable compounds.

That is, the present invention is as follows:
(1) A polymerizable compound having a lactone structure, the polymerizable compound being represented by the following formula (1): 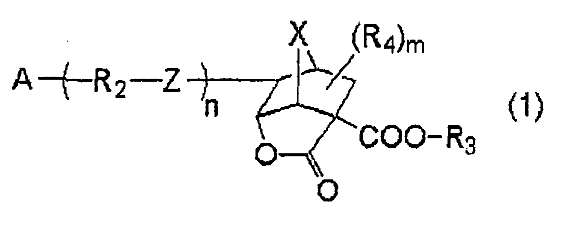 wherein
   A represents a polymerizable site;
   R₂ represents a single bond or a chain or cyclic alkylene group which may have a substituent, and when plural R₂s are present, R₂s may be the same as or different from every other R₂;
   R₃ represents a linear, branched or cyclic hydrocarbon group in which a part or all of hydrogen atoms linked to a constituent carbon atom or carbon atoms are substituted with a fluorine atom and which may further have a substituent;
   R₄ represents a halogen atom, a cyano group, a hydroxy group, an amide group, an alkyl group which may have a substituent, a cycloalkyl group which may have a substituent, an alkoxy group which may have a substituent, a phenyl group which may have a substituent, an acyl group which may have a substituent, an alkoxycarbonyl group which may have a substituent, or a group represented by R-C(=O)- or R-C(=O)O-, wherein R represents an alkyl group which may have a substituent or a cycloalkyl group which may have a substituent; and when plural R₄s are present, R₄s may be the same as or different from every other R₄, and two or more R₄s may be bonded to each other to form a ring,
   X represents an alkylene group which may have a substituent, an oxygen atom or a sulfur atom;
   Z represents a single bond, an ether bond, an ester bond, an amide bond, a urethane bond or a urea bond, and when plural Zs are present, Zs may be the same as or different from every other Z;
   n represents a repetition number and represents an integer of from 0 to 5; and
   m represents the number of substituents and represents an integer of from 0 to 7.
(2) The polymerizable compound as described in item (1) above, which is represented by the following formula (2): 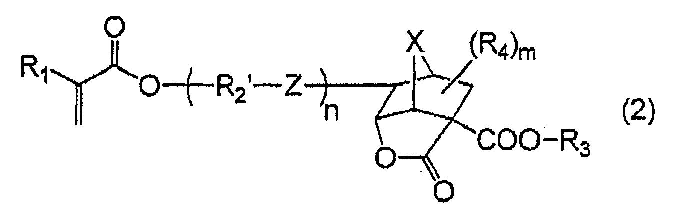 wherein
   R₁ represents a hydrogen atom, an alkyl group which may have a substituent, or a halogen atom; and
   R₂' represents a chain or cyclic alkylene group which may have a substituent, and when plural R₂s are present, R₂'s may be the same as or different from every other R'₂;
   R₃ represents a linear, branched or cyclic hydrocarbon group in which a part or all of hydrogen atoms linked to a constituent carbon atom or carbon atoms are substituted with a fluorine atom and which may further have a substituent;
   R₄ represents a halogen atom, a cyano group, a hydroxy group, an amide group, an alkyl group which may have a substituent, a cycloalkyl group which may have a substituent, an alkoxy group which may have a substituent, a phenyl group which may have a substituent, an acyl group which may have a substituent, an alkoxycarbonyl group which may have a substituent, or a group represented by R-C(=O)- or R-C(=O)O-, wherein R represents an alkyl group which may have a substituent or a cycloalkyl group which may have a substituent; and when plural R₄s are present, R₄s may be the same as or different from every other R₄, and two or more R₄s may be bonded to each other to form a ring;
   X represents an alkylene group which may have a substituent, an oxygen atom or a sulfur atom;
   Z represents a single bond, an ether bond, an ester bond, an amide bond, a urethane bond or a urea bond, and when plural Zs are present, Zs may be the same as or different from every other Z;
   n represents a repetition number and represents an integer of from 0 to 5; and
   m represents the number of substituents and represents an integer of from 0 to 7.
(3) The polymerizable compound as described in item (1) or (2) above, which is represented by the following formula (3): 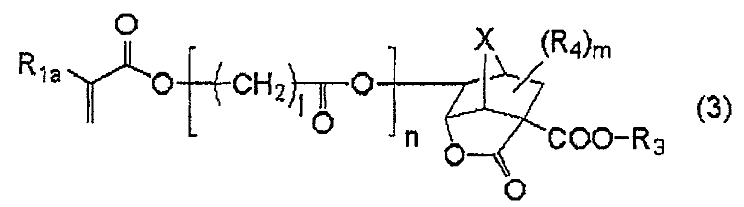 wherein
   R₁ₐ represents a hydrogen atom, a methyl group, a hydroxymethyl group, a halogenated methyl group or a halogen atom;
   R₃ represents a linear, branched or cyclic hydrocarbon group in which a part or all of hydrogen atoms linked to a constituent carbon atom or carbon atoms are substituted with a fluorine atom and which may further have a substituent;
   R₄ represents a halogen atom, a cyano group, a hydroxy group, an amide group, an alkyl group which may have a substituent, a cycloalkyl group which may have a substituent, an alkoxy group which may have a substituent, a phenyl group which may have a substituent, an acyl group which may have a substituent, an alkoxycarbonyl group which may have a substituent or a group represented by R-C(=4)- or R-C(=O)O-, wherein R represents an alkyl group which may have a substituent or a cycloalkyl group which may have a substituent; and when plural R₄s are present, R₄s may be the same as or different from every other R₄, and two or more R₄s may be bonded to each other to form a ring;
   X represents an alkylene group which may have a substituent, an oxygen atom or a sulfur atom;
   l represents a repetition number and represents an integer of from 1 to 5;
   n represents a repetition number and represents an integer of from 0 to 5; and
   m represents the number of substituents and represents an integer of from 0 to 7.
(4) The polymerizable compound as described in any of items (1) to (3) above,
   wherein, in the formulae (1) to (3), R₃ represents a linear hydrocarbon group in which a part or all of hydrogen atoms linked to a constituent carbon atom or carbon atoms are substituted with a fluorine atom, and
   the linear hydrocarbon group is a group represented by -(CH₂)ₙ-(CF₂)ₘ-CF₃, where n represents an integer of 0 or 1 and m represents an integer of from 0 to 10.
(5) The polymerizable compound as described in any of items (I) to (3) above,
   wherein, in the formulae (1) to (3), R₃ represents a branched hydrocarbon group in which a part or all of hydrogen atoms linked to a constituent carbon atom or carbon atoms are substituted with a fluorine atom, and
   the branched hydrocarbon group is a group represented by -C(Ra)(Rb)(Rc), where Ra, Rb and Rc each independently represents a hydrogen atom, an alkyl group, a cycloalkyl group or an aryl group provided that at least one of Ra, Rb and Rc represents an alkyl group having a fluorine atom as a substituent, a cycloalkyl group having a fluorine atom as a substituent or an aryl group having a fluorine atom as a substituent.
(6) The polymerizable compound as described in any of items (1) to (3) above,
   wherein, in the formulae (1) to (3), R₃ represents a cyclic hydrocarbon group in which a part or all of hydrogen atoms linked to a constituent carbon atom or carbon atoms are substituted with a fluorine atom, and
   the cyclic hydrocarbon group is a phenyl group having a fluorine atom as a substituent and which may further have a substituent, a cyclopentyl group having a fluorine atom as a substituent and which may further have a substituent or a cyclohexyl group having a fluorine atom as a substituent and which may further have a substituent.
(7) A lactone-containing compound represented by the following formula (4): 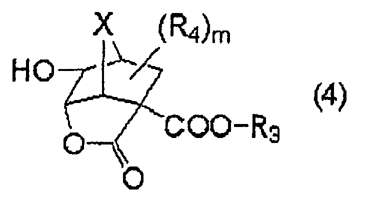 wherein
   R₃ represents a linear, branched or cyclic hydrocarbon group in which a part or all of hydrogen atoms linked to a constituent carbon atom or carbon atoms are substituted with a fluorine atom and which may further have a substituent;
   R₄ represents a halogen atom, a cyano group, a hydroxy group, an amide group, an alkyl group which may have a substituent, a cycloalkyl group which may have a substituent, an alkoxy group which may have a substituent, a phenyl groups which may have a substituent, an acyl group which may have a substituent, an alkoxycarbonyl group which may have a substituent or a group represented by R-C(=O)- or R-C(=O)O-, wherein R represents an alkyl group which may have a substituent or a cycloalkyl group which may have a substituent; and when plural R₄s are present, R₄s may be the same as or different from every other R₄, and two or more R₄s may be bonded to each other to form a ring;
   X represents an alkylene group which may have a substituent, an oxygen atom or a sulfur atom; and
   m represents the number of substituents and represents an integer of from 0 to 7.
(8) A method for manufacturing a lactone-containing compound represented by the following formula (4), the method including:
   reacting a carboxylic acid-containing compound represented by the following formula (5) with an alcohol represented by the following formula (6): 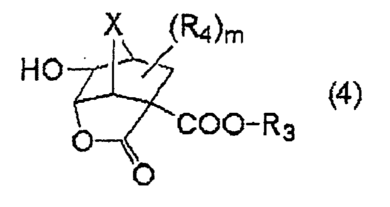 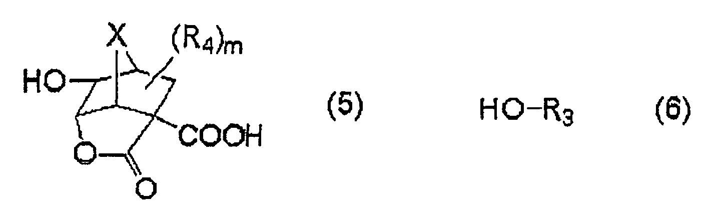 wherein
      R₃ represents a linear, branched or cyclic hydrocarbon group in which a part or all of hydrogen atoms linked to a constituent carbon atom or carbon atoms are substituted with a fluorine atom and which may further have a substituent;
      R₄ represents a halogen atom, a cyano group, a hydroxy group, an amide group, an alkyl group which may have a substituent, a cycloalkyl group which may have a substituent, an alkoxy group which may have a substituent, a phenyl group which may have a substituent, an acyl group which may have a substituent, an alkoxycarbonyl group which may have a substituent or a goup represented by R-C(=O)- or R-C(=O)O-, wherein R represents an alkyl group which may have a substituent or a cycloalkyl group which may have a substituent; and when plural R₄s are present, R₄s may be the same as or different from every other R₄, and two or more R₄s may be bonded to each other to form a ring;
      X represents an alkylene group which may have a substituents, an oxygen atom or a sulfur atom; and
      m represents the number of substituents and represents an integer of from 0 to 7.
(9) A polymer compound obtained by polymerizing the polymerizable compound as described in any of items (1) to (6) above.

The following embodiments are also preferable in the present invention.
(10) The lactone-containing compound as described in item (7) above,
   wherein, in the formula (4), R₃ represents a linear hydrocarbon group in which a part or all of hydrogen atoms linked to a constituent carbon atom or carbon atoms are substituted with a fluorine atom, and
   the linear hydrocarbon group is a group represented by -(CH₂)ₙ-(CF₂)ₘ-CF₃, where n represents an integer of 0 or 1 and m represents an integer of from 0 to 10.
(11) The lactone-containing compound as described in item (7) above,
   wherein, in the formula (4), R₃ represents a branched hydrocarbon group in which a part or all of hydrogen atoms linked to a constituent carbon atom or carbon atoms are substituted with a fluorine atom, and
   the branched hydrocarbon group is a group represented by-C(Ra)(Rb)(Rc), where Ra, Rb and Rc each independently represents a hydrogen atom, an alkyl group, a cycloalkyl group or an aryl group provided that at least one af Ra, Rb and Rc represents an alkyl group having a fluorine atom as a substituent, a cycloalkyl group having a fluorine atom as a substituent or an aryl groups having a fluorine atom as a substituent.
(12) The lactone-containing compound as described in item (7) above,
   wherein, in the formula (4), R₃ represents a cyclic hydrocarbon group in which, a part or all of hydrogen atoms linked to a constituent carbon atom or carbon atoms are substituted with a fluorine atom, and
   the cyclic hydrocarbon group is a phenyl group having a fluorine atom as a substituent and which may further have a substituent, a cyclopentyl group having a fluorine atom as a substituent and which may further have a substituent or a cyclohexyl group having a fluorine atom as a substituent and which may further have a substituent.
(13) The method for manufacturing a lactone-containing compound as described in item (8) above,
   wherein, in the formulae (4) and (6), R₃ represents a linear hydrocarbon group in which a part or all of hydrogen atoms linked to a constituent carbon atom or carbon atoms are substituted with a fluorine atom, and
   the linear hydrocarbon group is a group represented by -(CH₂)ₙ-(CF₂)ₘ-CF₃, where n represents an integer of 0 or 1 and m represents an integer of from 0 to 10.
(14) The method for manufacturing a lactone-containing compound as described in item (8) above,
   wherein, in the formulae (4) and (6), R₃ represents a branched hydrocarbon group in which a part or all of hydrogen atoms linked to a constituent carbon atom or carbon atoms are substituted with a fluorine atom, and
   the branched hydrocarbon group is a group represented by -C(Ra)(Rb)(Rc), where Ra, Rb and Rc each independently represents a hydrogen atom, an alkyl group, a cycloalkyl group or an aryl group provided that at least one of Ra, Rb and Rc represents an alkyl group having a fluorine atom as a substituent, a cycloalkyl group having a fluorine atom as a substituent or an aryl group having a fluorine atom as a substituent.
(15) The method for manufacturing a lactone-containing compound as described in item (8) above,
   wherein, in the formulae (4) and (6), R₃ represents a cyclic hydrocarbon group in which a part or all of hydrogen atoms linked to a constituent carbon atom or carbon atoms are substituted with a fluorine atom, and
   the cyclic hydrocarbon group is a phenyl group having a fluorine atom as a substituent and which may further have a substituent, a cyclopentyl group having a fluorine atom as a substituent and which may further have a substituent or a cyclohexyl group having a fluorine atom as a substituent and which may further have a substituent.

### BRIEF DESCRIPTION OF THE DRAWINGS

The features of the invention will appear more fully upon consideration of the exemplary embodiments of the invention, which are schematically set forth in the drawing, in which:
Fig. 1 is an NMR chart of Compound (2) synthesized in the Examples;
Fig. 2 is an NMR chart of Compound (3) synthesized in the Examples;
Fig. 3 is an NMR chart of Compound (4) synthesized in the Examples;
Fig. 4 is an NMR chart of Compound (8) synthesized in the Examples;

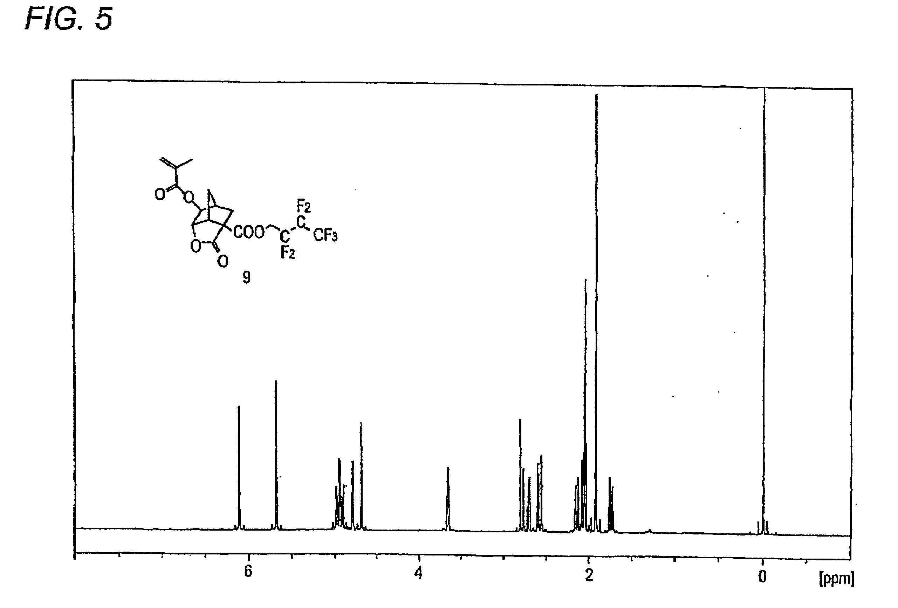

Fig. 5 is an NMR chart of Compound (9) synthesized in the Examples;
Fig. 6 is an NMR chart of Compound (10) synthesized in the Examples; and
Fig. 7 is an NMR chart of Compound (11) synthesized in the Examples.
Fig. 8 is an NMR chart of Compound (19) synthesized in the Examples.
Fig. 9 is an NMR chart of Compound (22) synthesized in the Examples,

### DETAILED DESCRIPTION OF THE INVENTION

Best modes for carrying out the invention are hereunder described.

In the expressions of groups (atomic groups) in this specification, an expression which does not express "substituted" or "unsubstituted" includes both one not having a substituent and one having a substituent. For example, the "alkyl group" includes not only an alkyl group not having a substituent (unsubstituted alkyl group) but an alkyl group having a substituent (substituted alkyl group),

Polymerizable compound having a lactone structure represented by the formula (1):

The invention is concerned with a polymerizable compound having a lactone structure represented by the following formula (1).

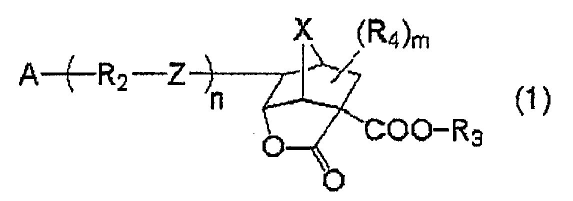

In the formula (1),
A represents a polymerizable site;
R₂ represents a single bond or a chain or cyclic alkylene group which may have a substituent, and when plural R₂s are present, R₂s may be the same as or different from every other R₂;
R₃ represents a linear, branched or cyclic hydrocarbon group in which a part or all of hydrogen atoms linked to a constituent carbon atom or carbon atoms are substituted with a fluorine atom and which may further have a substituent;
R₄ represents a halogen atom, a cyano group, a hydroxy group, an amide group, an alkyl group which may have a substituent, a cycloalkyl group which may have a substituent, an alkoxy group which may have a substituent, a phenyl group which may have a substituent, an acyl group which may have a substituent, an alkoxycarbonyl group which may have a substituent or a group represented by R-C(=O)- or R-C(=O)O-, wherein R represents an alkyl group which may have a substituent or a cycloalkyl group which may have a substituent; and when plural R₄s are present, R₄s may be the same as or different from every other R₄, and two or more R₄s may be bonded to each other to form a ring;
X represents an alkylene group which may have a substituent, an oxygen atom or a sulfur atom;
Z represents a single, bond, an ether bond, an ester bond, an amide bond, a urethane bond or a urea bond, and when plural Zs are present, Zs may be the same as or different from every other Z;
n represents a repetition number and represents an integer of from 0 to 5; and
m represents the number of substituents and represents an integer of from 0 to 7.

The polymerizable site represented by A is not particularly limited, but it is preferably a skeleton having a group capable of undergoing radical polymerization, anionic polymerization or cationic polymerization. The polymerizable site represented by A is more preferably a (meth)acrylate skeleton, a styrene skeleton, an epoxy skeleton or a norbornene skeleton. These skeletons may have a substituent. Examples of the substituent thereof include halogen atoms (for example, a fluorine atom), halogenated alkyl groups (for example, trifluoromethyl), hydroxyl group or alkoxy groups such as methoxy group, ethoxy group, isopropoxy group, tert-butoxy group, benzyloxy group or the like. As the polymerizable site represented by A, a (meth)acrylate skeleton is especially preferable.

R₂ is preferably a chain alkylene group or a cyclic alkylene group. The chain alkylene group is preferably a chain alkylene group having from 1 to 10 carbon atoms, and more preferably from 1 to 5 carbon atoms, and still more preferably from 1 to 3 carbon atoms, and examples thereof include a methylene group, an ethylene group, a propylene group and an isopropylene group. The cyclic alkylene group is preferably a cyclic alkylene group having from 3 to 20 carbon atoms, and examples thereof include a cyclohexylene group, a cyclopentylene group, a norbomylene group and an adamantylene group. The group represented by R₂ is more preferably a chain alkylene group. Each of the chain alkylene groups and the cyclic alkylene groups is not particularly limited and may have a substituent. Examples of the substituent on each of the chain alkylene groups and the cyclic alkylene groups include, for example, a halogen atom such as a fluorine atom, a chlorine atom, a bromine atom; a mercapto group; a hydroxy group; an alkoxy group such as a methoxy group, an ethoxy group, an isopropoxy group, a t-butoxy group, a benzyloxy group; an alkyl group such as a methyl group, an ethyl group, a propyl group, an isopropyl group, a butyl group, a sec-butyl group, a t-butyl group, a pentyl group, a hexyl group; a cycloalkyl group such as a cyclopropyl group, a cyclobutyl group, a cyclopentyl group, a cyclohexyl group, a cycloheptyl group; a cyano group; a nitro group; a sulfonyl group; a silyl group, an ester group; an acyl group; a vinyl group; and an aryl croup. In the case where the foregoing n is 2 or more, plural groups represented by R₂ may be the same as or different from every other R₂.

The group represented by R₃ is not particularly limited so far as it is a linear, branched or cyclic hydrocarbon group in which a part or all of hydrogen atoms linked to a constituent carbon atom or carbon atoms are substituted with a fluorine atom each of which may further have a substituent. The number of carbon atoms is preferably from 1 to 10, and more preferably from 3 to 5.

Examples of the substituent which the linear, branched or cyclic hydrocarbon group may further have include a halogen atom other than a fluorine atom, a mercapto group, a hydroxyl group, an alkoxy group (for example, a methoxy goup, an ethoxy group, an isopropoxy group, a t-butoxy group, a benzyloxy group, etc.), an alkyl group (for example, a methyl group, an ethyl group, a propyl group, an isopropyl group, a butyl group, a sec-butyl group, a t-butyl group, a pentyl group, a hexyl group, etc.), a cycloalkyl group (for example, a cyclopropyl group, a cyclobutyl group, a cyclopentyl group, a cyclohexyl group, a cycloheptyl group, etc.), a cyano group, a nitro group, a sulfonyl group, a silyl group, an ester group, an acyl group, a vinyl group and an aryl group. Of these, an alkyl group is especially preferable.

In the case where the linear, branched or cyclic hydrocarbon group further has a substituent, the "linear, branched or cyclic hydrocarbon group in which a part or all of hydrogen linked to a constituent carbon atom or carbon atoms are substituted with a fluorine atom and which may further have a substituent" for R₃ includes not only a "group in which a part or all of hydrogen atoms in the linear, branched or cyclic hydrocarbon group are substituted with a fluorine atom" but a "group in which apart or all of hydrogen atoms of a further substituent which the linear, branched or cyclic hydrocarbon group has are substituted with a fluorine, atom".

The number of fluorine atoms is preferably from 1 to 15, and more preferably from 2 to 9.

The linear hydrocarbon group as R₃, in which a part or all of hydrogen atoms linked to a constituent carbon atom or carbon atoms are substituted with a fluorine atom, is preferably a group represented by -(CH₂)ₙ-(CF₂)ₘ-CF₃ (wherein n represents 0 or 1; and m represents an integer of from 0 to 10). m is preferably an integer of from 1 to 7, and more preferably an integer of from 2 to 5.

Examples of the branched hydrocarbon group as R₃, in which a part or all of hydrogen atoms linked to a constituent carbon atom or carbon atoms are substituted with a fluorine atom, include a group represented by -C(Ra)(Rb)(Rc), wherein each of Ra, Rb and Rc independently represents a hydrogen atom, an alkyl group, a cycloalkyl group or an aryl group, and at least any one of them is an alkyl group, a cycloalkyl group or an aryl group each having a fluorine atom as a substituent, with an alkyl group having a fluorine atom as a substituent being more preferable (for example, 1,1,1,3,3,3-hexafluoroisopropyl group is preferable). Incidentally, it is preferable that two of Ra, Rb and Rc each does not represent a hydrogen atom.

Examples of the cyclic hydrocarbon group as R₃, in which a part or all of hydrogen atoms linked to a constituent carbon atom or carbon atoms are substituted with a fluorine atom, include a phenyl group, cyclopentyl group and a cyclohexyl group each having a fluorine atom as a substituent, with phenyl group having a fluorine atom as a substituent being preferable. Here, for example, each of the phenyl group, the cyclopentyl group and the cyclohexyl group may further have a substituent, and as described previously, this further substituent may be substituted with a fluorine atom. Examples of the substituent which each of the phenyl group, the cyclopentyl group and the cyclohexyl group may further have include the specific examples exemplified above as the substituent which the linear, branched or cyclic hydrocarbon group may further have. Specific examples of the preferred cyclic hydrocarbon group as R₃, in which a part or all of hydrogen atoms linked to a constituent carbon atom or carbon atoms are substituted with a fluorine atom, include a pentafluorophenyl group, a 2,3,5,6-tetrafluoro-4-(trifluoromethyl)phenyl group a 2,2,3,3-tetrafluorocyclopentyl group and pentafluorocyclohexyl group.

Each of Ra, Rb and Rc is preferably an alkyl group; and the fluorine atom is preferably present as a trifluoromethyl group.

In the formula (I), when the ester bond in the lactone group and -COOR₃ are located adjacent to each other, the hydrolyzability is enhanced, and the affinity with a developing solution is enhanced.

The alkyl group as R₄ is preferably a linear alkyl group or a branched chain alkyl group each having from 1 to 30 carbon atoms, and more preferably from 1 to 15 carbon atoms. Specific examples thereof include linear alkyl groups such as a methyl group, an ethyl group, an n-propyl group, an n-butyl group, an n-pentyl group, an n-hexyl group, an n-octyl group, an n-dodecyl group, an n-tetradecyl group, an n-octadecyl group; and branched alkyl groups such as an isopropyl group, an isobutyl group, a t-butyl group, a neopentyl group, a 2-ethylhexyl group.

The cycloalkyl group as R₄ is preferably a cycloalkyl group having from 3 to 20 carbon atoms. The cycloalkyl group may be polycyclic and may have an oxygen atom within a ring thereof. Specific examples thereof include a cyclopropyl group, a cyclopentyl group, a cyclohexyl group, a norbornyl group and an adamantyl group.

The alkoxy group as R₄ is preferably an alkoxy group having from 1 to 30 carbon atoms; and examples of the alkyl group in the alkoxy group include the foregoing alkyl groups.

The acyl group as R₄ is preferably an acyl group having from 2 to 30 carbon atoms; and examples of the alkyl group in the acyl group include the foregoing alkyl groups.

The alkoxycarbonyl group as R₄ is preferably an alkoxycarbonyl group having from 2 to 30 carbon atoms; and examples of the alkyl group in the alkoxycarbonyl group include the foregoing alkyl groups. R in the group represented by R-C(=O)- or R-C(=O)O- as R₄ represents an alkyl group which may have a substituent or a cycloalkyl group which may have a substituent.

Each of the alkyl group, the cycloalkyl group, the alkoxy group, the phenyl group, the acyl group and the alkoxycarbonyl group as R₄ may have a substituent. Examples of the substituent on each of the alkyl group, the cycloalkyl group, the alkoxy group, the phenyl group, the acyl group and the alkoxycarbonyl group include a halogen atom such as a fluorine atom, a chlorine atom, a bromine atom; a mercapto group; a hydroxy group; an alkoxy group such as a methoxy group, an ethoxy group, an isopropoxy group, a t-butoxy group, a benzyloxy group; an alkyl group such as a methyl group, an ethyl group, a propyl group, an isopropyl group, a butyl group, a sec-butyl group, a t-butyl group, a pentyl group, a hexyl group; a cycloalkyl group such as a cyclopropyl group, a cyclobutyl group, a cyclopentyl group, a cyclohexyl group, a cycloheptyl group; a cyano group; a nitro group; a sulfonyl group; a silyl group; an ester group; an acyl group; a vinyl group; and an aryl group. Also, in a case where plural R₄s are present, the groups represented by R₄ may be bonded to each other to form a cycloalkylene group.

The alkyl group having a substituent as R₄ includes a halogenated alkyl group, and examples thereof include the foregoing alkyl groups in which at least a part of hydrogen atoms is substituted with a halogen atom. These alkyl groups may further have a substituent.

The cycloalkyl group having a substituent as R₄ is preferably a halogenated cycloalkyl group, and examples thereof include the foregoing cycloalkyl groups in which at least a part of hydrogen atoms is substituted with a halogen atom. These cycloalkyl groups may further have a substituent.

The alkoxy group having a substituent as R₄ is preferably a halogenated alkoxy group, and examples thereof include the foregoing alkoxy groups in which at least a part of hydrogen atoms is substituted with a halogen atom. These alkoxy groups may further have a substituent.

The phenyl group having a substituent as R₄ includes halogenated phenyl groups in which at least a part of hydrogen atoms in the phenyl group is substituted with a halogen atom. These phenyl groups may further have a substituent,

The acyl group having a substituent as R₄ is preferably a halogenated acyl group, and examples thereof include the foregoing acyl groups in which at least a part of hydrogen atoms is substituted with a halogen atom. These acyl groups may further have a substituent,

The alkoxycarbonyl group having a substituent as R₄ is preferably a halogenated alkoxycarbonyl group, and examples thereof include the foregoing alkoxycarbonyl groups in which at least a part of hydrogen atoms is substituted with a halogen atom. These alkoxycarbonyl groups may further have a substituent. That is, the alkyl group in the alkoxycarbonyl group as R₄ may be a halogenated alkyl group, and examples thereof as R₄ include (CF₃)₂HCOC(=O)-.

Examples of the substituent which each of the alkyl group having a substituent, the cycloalkyl group having a substituent, the alkoxy group having a substituent, the phenyl group having a substituent, the acyl group having a substituent and the alkoxycarbonyl group having a substituent as R₄ may further have include the same substituents as those which each of the alkyl group, the cycloalkyl group, the alkoxy goup, the phenyl group, the acyl group and the alkoxycarbonyl group as R₄ may have.

Preferred Examples of the alkyl group, the alkyl group having a substituent, the cycloalkyl group and the cycloalkyl group having a substituent as R are the same as those described for the alkyl group, the alkyl group having a substituent, the cyclyalkyl group and the cycloalkyl group having a substituent as R₄, respectively.

X represents an alkylene group which may have a substituent, an oxygen atom or a sulfur atom. The alkylene group which may have a substituent is preferably an alkylene group having from 1 to 2 carbon atoms, and example thereof include a methylene group and an ethylene group, with methylene group being especially preferable. Examples of the substituent on the alkylene group include a halogen atom such as a fluorine atom, a. chlorine atom, a bromine atom; a mercapto group; a hydroxy group; an alkoxy group such as a methoxy group, an ethoxy group, an isopropoxy group, a t-butoxy group, a benzyloxy group; an alkyl group such as a methyl group, an ethyl group, a propyl group, an isopropyl group, a butyl group, 8 sec-butyl group, a t-butyl group, a pentyl group, a hexyl group; a cycloalkyl group such as a cyclopropyl group, a cyclobutyl group, a cyclopentyl group, a cyclohexyl group, a cycloheptyl group; a cyano group; a nitro group; a sulfonyl group; a silyl group; an ester group; an acyl group; a vinyl group; and an aryl group.

Examples of the group represented by Z include a single bond, an ether bond, an ester bond, an amide bond, a urethane bond and a urea bond Of these, a single bond, an ether bond and an ester bond are preferable, with an ester bond being especially preferable. Z may be located on any of an endo side or an exo side of the norbornane skeleton.

n represents a repetition number and represents an integer of from 0 to 5, preferably an integer of from 0 to 2, and more preferably an integer of 0 or 1. n is more preferably 0.

m represents the number or substituents and represents an integer of from 0 to 7, preferably an integer of from 0 to 5, and especially preferably an integer of from 0 to 3. m is most preferably 0.

The polymerizable compound having a lactone structure represented by the formula (I) is preferably represented by the following formula (2).

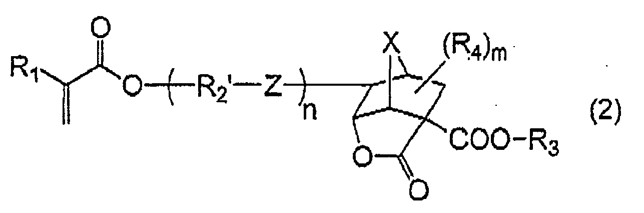

In the formulae (2), R₁ represents a hydrogen atom, an alkyl group which may have a substituent or a halogen atom.

R₂' represents a chain or cyclic alkylene group which may have a substituent, and when plural R₂'s are present, R₂'s may be the same as or different from every other R₂'

In the formula (2), R₃, R₄, X, Z, n and m are synonymous with R₃, R₄, X, Z, n and m in the foregoing formula (I).

Examples of the group represented by R₁ include a hydrogen atom, an alkyl group which may have a substituent and a halogen atom. R₁ is preferably a hydrogen atom or an alkyl group which may have a substituent (preferably having from 1 to 5 carbon atoms). Examples of the preferred substituent on the alkyl group include a halogen atom, a hydroxy group and an alkoxy group such as a methoxy group, an ethoxy group, an isopropoxy group, a t-butoxy group, a benzyloxy group.

The group as R₁ is most preferably a hydrogen atom, a methyl group, a hydroxymethyl group or a trifluoromethyl group.

Preferred examples and specific examples of the chain or cyclic alkylene group as R₂' are the same as those described for the chain or cyclic alkylene group which may have a substituent as R₂ in the formula (1).

The polymerizable compound having a lactone structure represented by the formula (1) is preferably represented by the following formula (3).

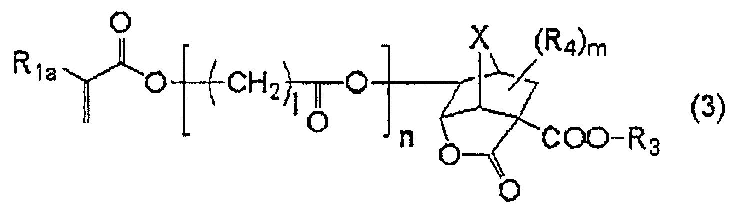

In the formulae (3),
R₁ₙ represents a hydrogen atom, a methyl group, a hydroxymethyl group, a halogenated methyl group or a halogen atom; and
l represents a repetition number and represents an integer of from 1 to 5.

In the formulae (3), R₃, R₄, X, n and m are synonymous with R₃, R₄, X, n and m in the formula (1).

l represents a repetition number of a methylene group and represents an integer of from 1 to 5, and preferably an integer of from 1 to 3.

Though a synthesis method of the compound represented by the formula (1) is not particularly limited, the compound represented by the formula (1) can be synthesized in the following method.

That is, a corresponding cyanolactone is hydrolyzed and the cyano group thereof is converted into a carboxylic acid thereby carboxylic acid-containing compound represented by the formula (5) is obtained. After that the carboxylic acid-containing compound represented by the formula (5) is allowed to react with an alcohol represented by the formula (6), thereby obtaining a compound represented by the formula (4). It has been newly found that in this esterification reaction, a hydroxy group of the carboxylic acid-containing compound represented by the formula (5) does not react, but the carboxylic acid as the carboxylic acid-containing compound represented by the formula (5) selectively reacts with the hydroxyl group of the alcohol represented by the formula (6), whereby the compound represented by the formula (4) is obtained in a stoichiometric yield.

The foregoing esterification reaction can be easily carried out under a general condition. For example, the esterification reaction is preferably carried out by successively or simultaneously adding the carboxylic acid-containing compound represented by the formula (5), the alcohol represented by the formula (6) and a condensing agent such as a carbodiimide-containing compound (for example, 1-ethyl-3-(3-dimethylaminopropyl)carbodiimide hydrochloride, N,N'-dicyclohexylcarbodiimide, N-(tert-butyl)-N'-ethylcarbodiimide, N;N'-di(tert-butyl)carbodiimide, etc.) and further, usually a base such as 4-dimethylaminopyridine in a solvent (for example, chloroform, tetrahydrofuran, dichloroethane, ethyl acetate, acetonitrile, etc,), wherein the reaction is preferably carried out, usually at a room temperature of 25°C, or upon cooling or heating or the like, if desired.

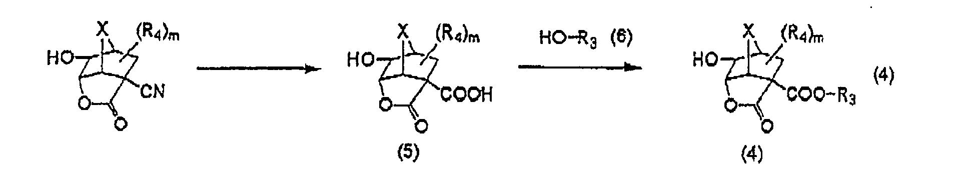

In the formulae (4) to (6) in the above scheme, R₃, R₄, X and m are synonymous with R₃, R₄, X, and m in the formula (1).

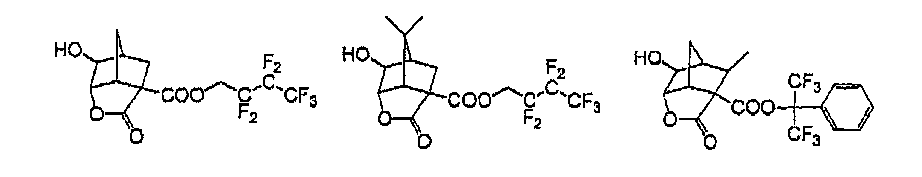

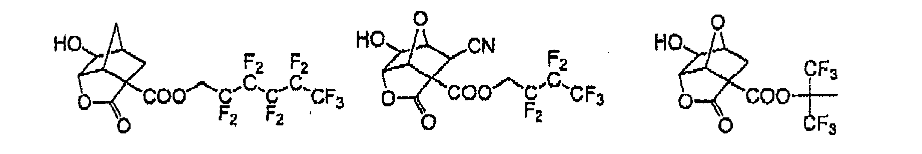

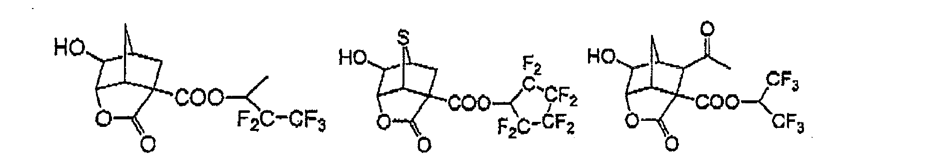

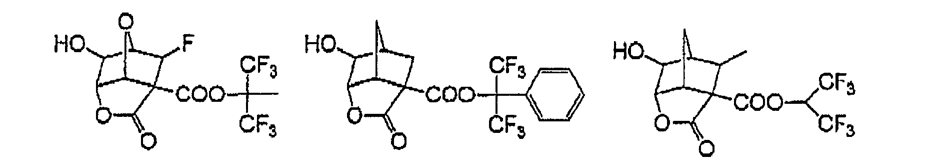

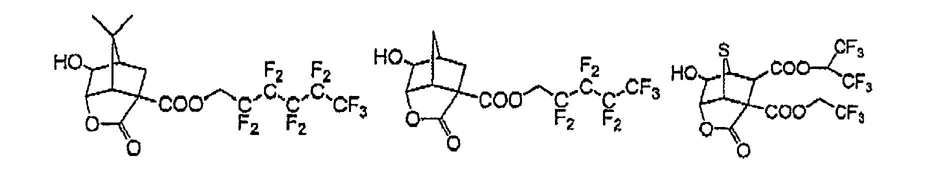

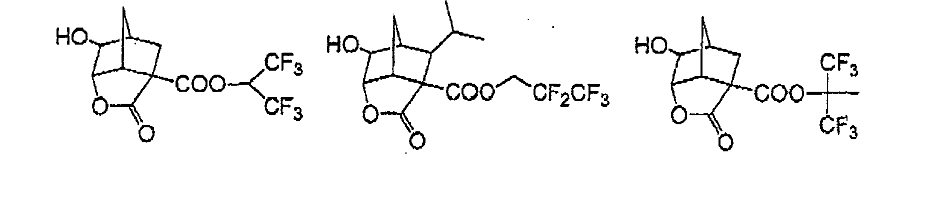

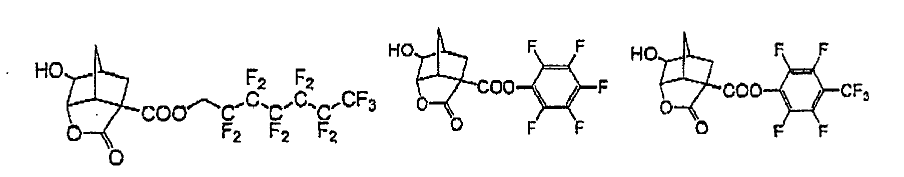

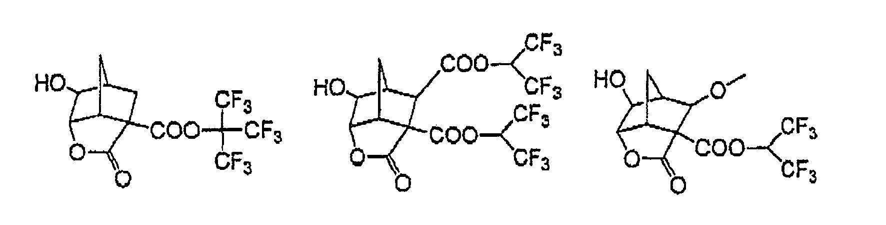

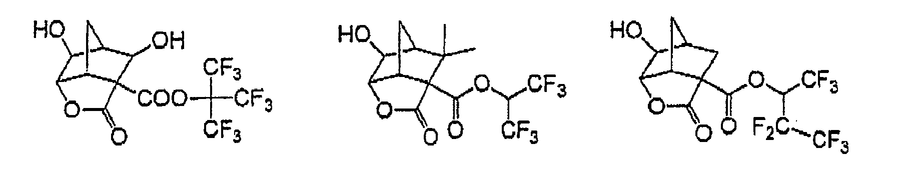

Specific examples of the lactose-containing compound represented by the formula (4) are described below, but it should not be construed that the invention is limited thereto.

A polymerizable site A and a spacer site (-R₂-Z-), where A, R₂ and Z are synonymous with A, R₂ and Z in the formula (1), if desired can be introduced by directly connecting a unit having a polymerizable site, or a polymerizable site and a spacer site to the hydroxyl group of the compound represented by the formula (4).

The introduction of the polymerizable site A, or the polymerizable site and the spacer site into the hydroxy group of the compound represented by the formula (4) can be easily carried out by a known method.

In the case where the end of each of the polymerizable site A and the spacer site is an acid chloride, the esterification reaction may be carried out by successively or simultaneously adding the compound represented by the formula (4), an acid chloride corresponding to the polymerization site or the spacer site (for example, methacrylic acid chloride, norbornene carboxylic acid chloride, etc.) and a base (for example, triethylamine, pyridine, 4-dimethylaminopyridine, etc.) in a solvent (for example, tetrahydrofuran, acetonitrile, ethyl acetate, diisopropyl ether, methyl ethyl ketone, etc.), thereby allowing the mixture to react upon cooling or heating or the like, if desired,

In the case where the end of each of the polymerizable site A and the spacer site has a carboxylic acid, the esterification reaction may be carried out by adding the compound represented by the formula (4), a carboxylic acid corresponding to the polymerizable site A or the spacer site (for example, methacrylic acid, norbornene carboxylic acid, etc.) and an inorganic acid (for example, hydrochloric acid, sulfuric-acid, nitric acid, perchloric acid, etc.) or an organic acid (for example, p-toluenesulfonic acid, benzenesulfonic acid, etc.), thereby achieving the reaction upon heating and optionally removing generated water from the system, etc.

Specific examples of the polymerizable compound having a lactone structure will be hereunder given, but it should not be construed that the invention is limited thereto.

In the following formulae, R₁ represents a hydrogen atom, an alkyl group which may have a substituent or a halogen atom. R₁ preferably represents a hydrogen atom, a methyl group, a hydroxymethyl group, a trifluoromethyl group or a halogen atom.

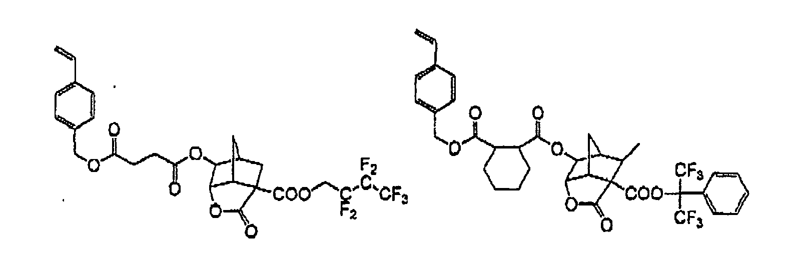

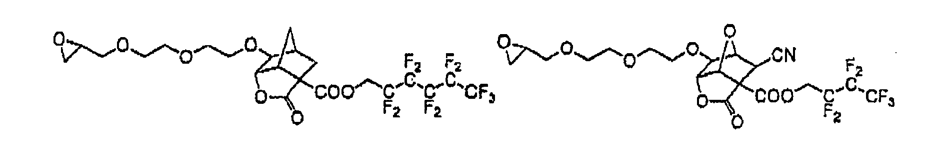

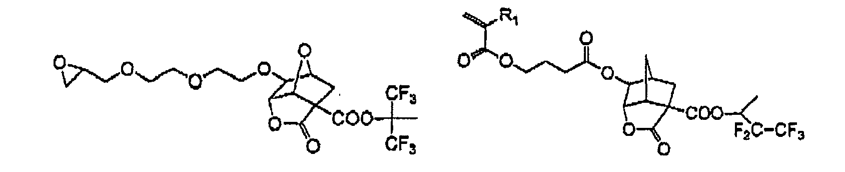

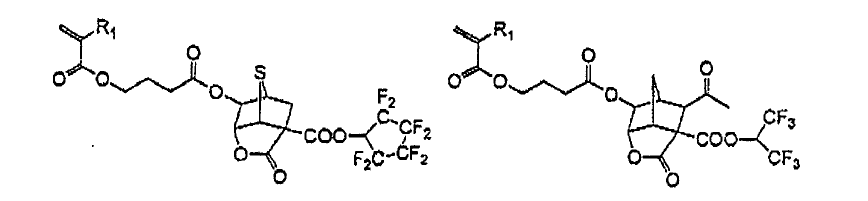

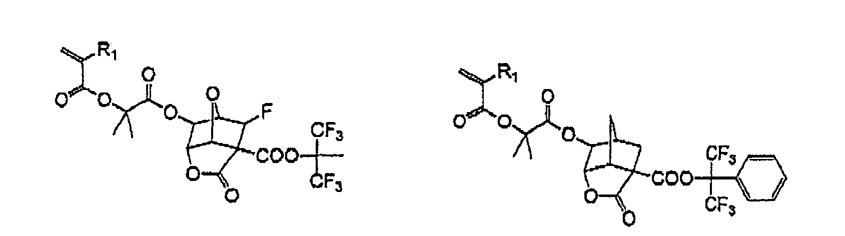

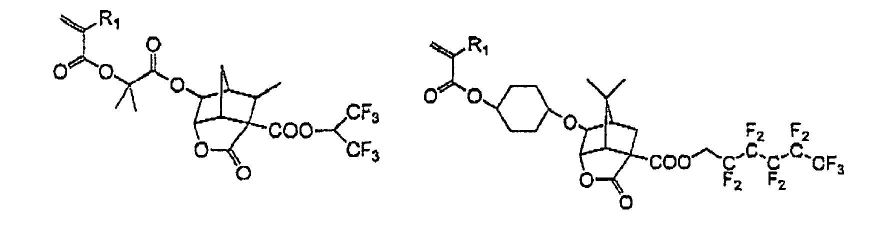

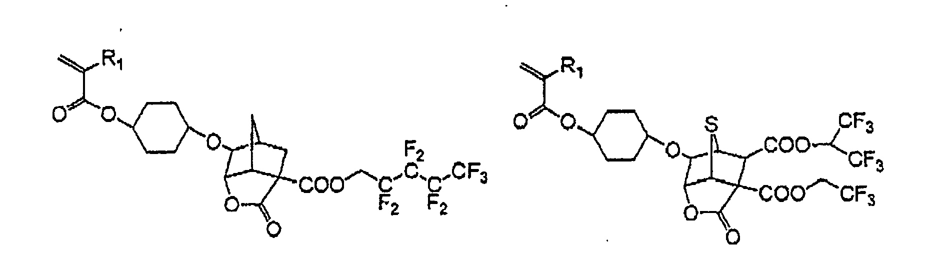

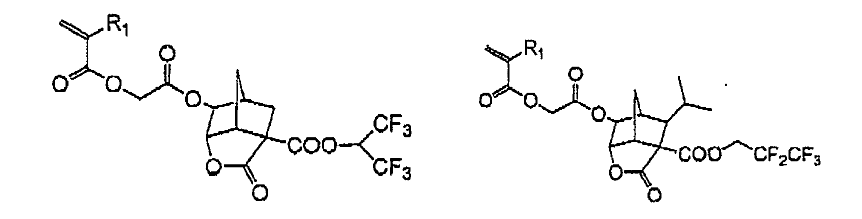

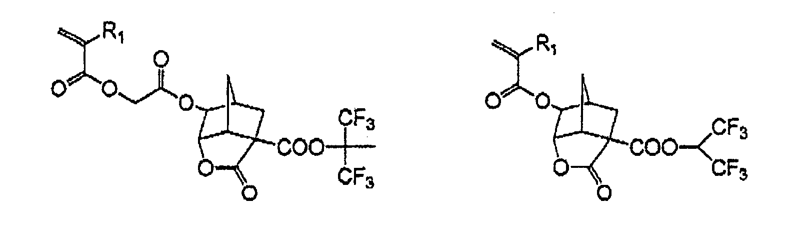

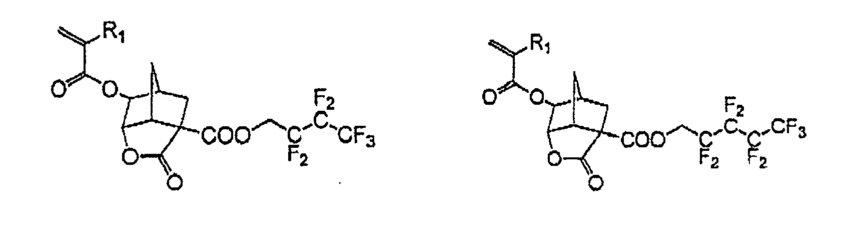

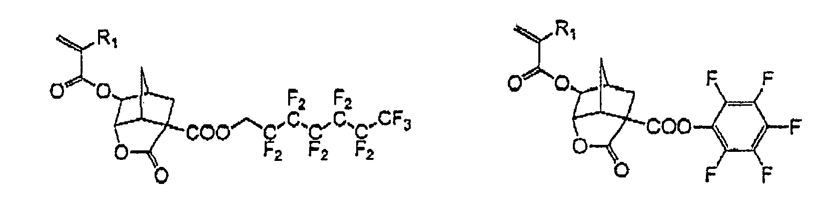

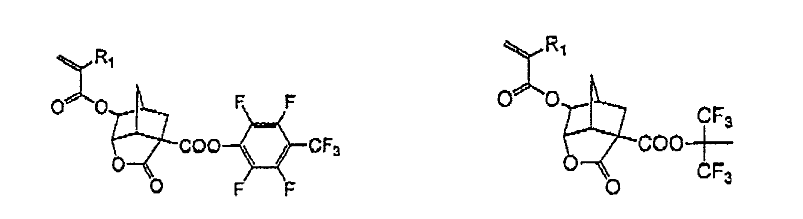

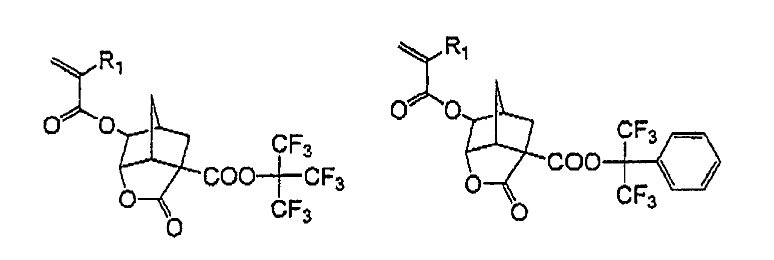

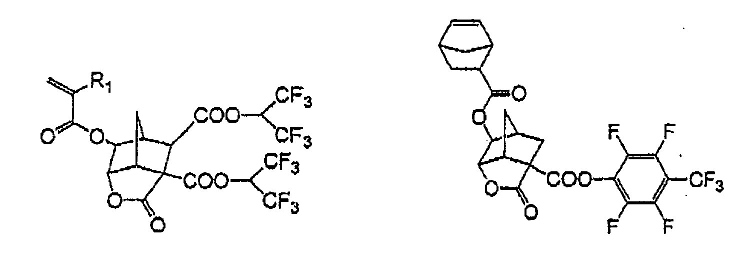

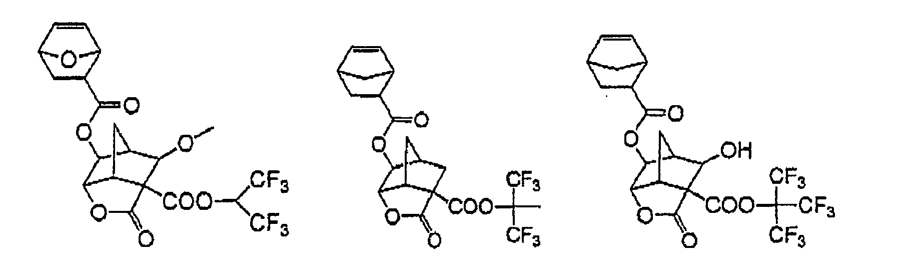

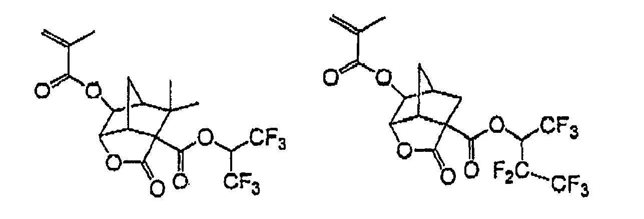

Polymer compound having a repeating unit corresponding to the polymerizable compound having a lactone structure represented by the formula (1):

The polymer compound having a repeating unit corresponding to the polymerizable compound having a lactone structure represented by the formula (1) contributes to an enhancement of the developability by an alkaline developing solution in the formation of a pattern in the photoresist field. By adding the present polymer compound to a resist, the developability by an alkaline developing solution can be enhanced. The present polymer compound may also be a resin containing not only a repeating unit corresponding to the polymerizable compound having a lactone structure represented by the formula (1) but a repeating unit having a group which is decomposed by the action of an acid to generate an alkali-soluble group (a resin which is decomposed by the action of an acid to become soluble in an alkaline developing solution, i.e., a so-called acid-decomposable resin).

A weight average molecular weight by GPC (gel permeation chromatography) method as reduced into standard polystyrene of the polymer compound of the invention is preferably from 1,000 to 100,000, more preferably from 1,000 to 50,000, and further preferably from 2,000 to 15,000.

The polymer compound of the invention can be obtained by polymerizing the foregoing compounds of the present invention (lactone structure-containing polymerizable compound represented by the formula (1)) according to the usual way (for example, radical polymerization). Examples of a general synthesis method include a batch polymerization method in which a monomer species and an initiator are dissolved in a solvent, and the solution is heated for polymerization; and a dropping polymerization method in which a solution of a monomer species and an initiator is added dropwise to a heated solvent over from I to 10 hours. Of these, a dropping polymerization method is preferable. Examples of the reaction solvent include ethers (for example, tetrahydrofuran, 1,4-dioxane, diisopropyl ether, etc.); ketones (for example, methyl ethyl ketone, methyl isobutyl ketone, etc.); ester solvents (for example, ethyl acetate); amide solvents (for example, dimethylformamide, dimethylacetamide, etc.); and solvents such as propylene glycol monomethyl ether acetate (PGMEA, also known as 1-methoxy-2-acetoxypropane), propylene glycol monomethyl ether (PGME, also known as 1-methoxy-2-propanol), cyclohexanone as described later.

The polymerization reaction is preferably carried out in an inert gas atmosphere such as nitrogen and argon. As to the polymerization initiator, the polymerization is started using a commercially available radical initiator (for example, an azo based initiator, a peroxide, etc.). The radical initiator is preferably an azo based initiator; and an azo based initiator having an ester group, a cyano group or a carboxyl group is preferable. Examples of the preferred initiator include azobisisobutyronitrile, azobisdimethylvaleronitrile and dimethyl 2,2'-azobis(2-methylpropionate). The concentration of the reaction solution is from 5 to 50 % by mass, and preferably from 30 to 50 % by mass. The reaction temperature is usually from 10°C to 150°C, preferably from 30°C to 120°C, and more preferably from 60°C to 100°C.

A resist composition (for examples positive working resist composition) using the polymer compound of the invention may be prepared by, for example, dissolving the polymer compound of the invention, a compound capable of generating an acid upon irradiation with actinic rays or radial rays and if desired, a surfactant, a basic compound, etc. in an organic solvent, followed by filtration through a filter.

The invention is hereunder described with reference to the following Examples, but it should not be construed that the invention is limited thereto.

### Example 1 (Synthesis of Compound (4))

The following Compound (1) was synthesized by a method disclosed in WO 07/037213.

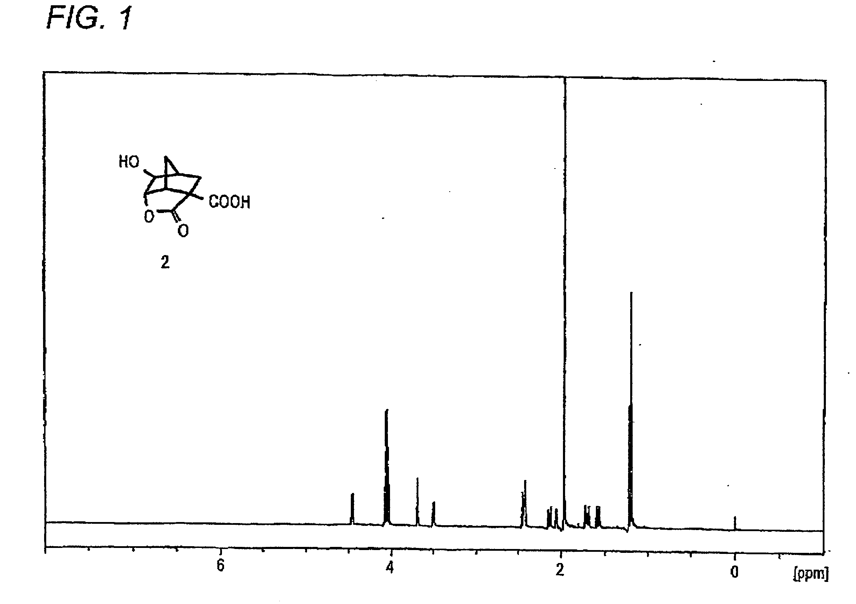

To 35.00 g of Compound (1), 150.00 g of water was added, and 27.30 g of NaOH was further added, The mixture was stirred for 9 hours under a heating and reflux condition. The reaction mixture was made acidic by the addition of hydrochloric acid and then extracted with ethyl acetate. Organic layers were concentrated to obtain 36.90 g of Compound (2) shown below (yield: 93 %). A ¹H-NMR chart of Compound (2) (unpurified) is shown in Fig. 1.

¹H-NMR (400 MHz in (CD₃)₂CO): σ (ppm)= 1.56 to 1.59 (1H), 1.68 to 1.72 (1H), 2.13 to 2.15 (1H), 2.13 to 2.47 (2H), 3.49 to 3.51 (1H), 3.68 (1H), 4.45 to 4.46 (1H)

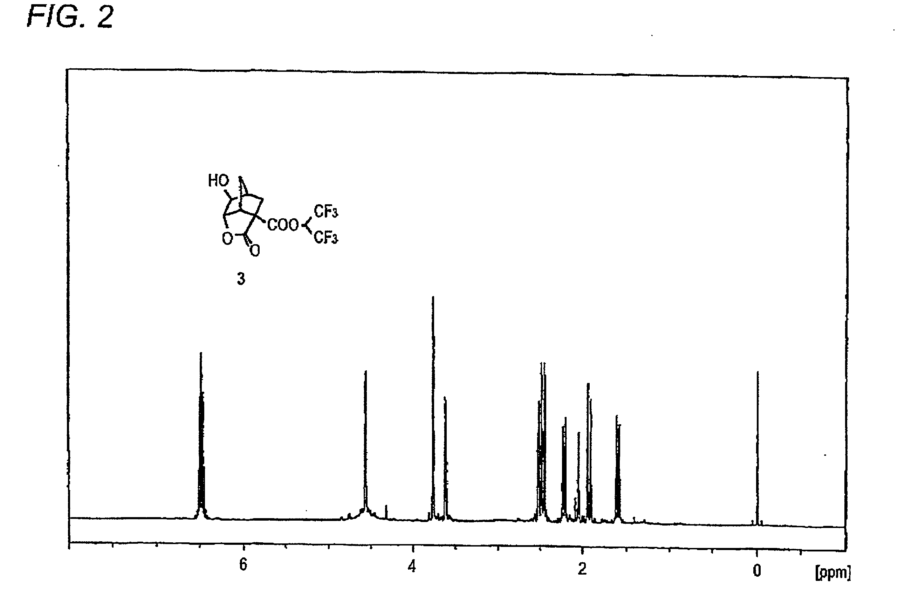

To 20.00 g of Compound (2), 200 mL of CHCl₃ was added, and 50.90 g of 1,1,1,3,3,3-hexafluoroisopropyl alcohol and 30.00 g of 4-dimethylaminopyridine were further added, followed by stirring. 22.00 g of 1-ethyl-3-(3-dimethylaminopropyl)carbodiimide hydrochloride was added to the resulting solution, and the mixture was stirred for 3 hours. The reaction solution was added to 500 mL of 1 N HCl, thereby stopping the reaction. An organic layer was further washed with 1 N HCl and subsequently washed with water, and the organic layer was concentrated to obtain 30.00 g of Compound (3) shown below (yield; 85 %). A ¹H-NMR chart of Compound (3) is shown in Fig. 2.

¹H-NMR (400 MHz in (CD₃)₂CO): σ (ppm) = 1.62 (1H), 1.91 to 1.95 (1H), 2.21 to 2.24 (1H), 2,45 to 2.53 (2H), 3.61 to 3.63 (1H), 3.76 (1H), 4.32 to 4.58 (1H), 6.46 to 6.53 (1H)

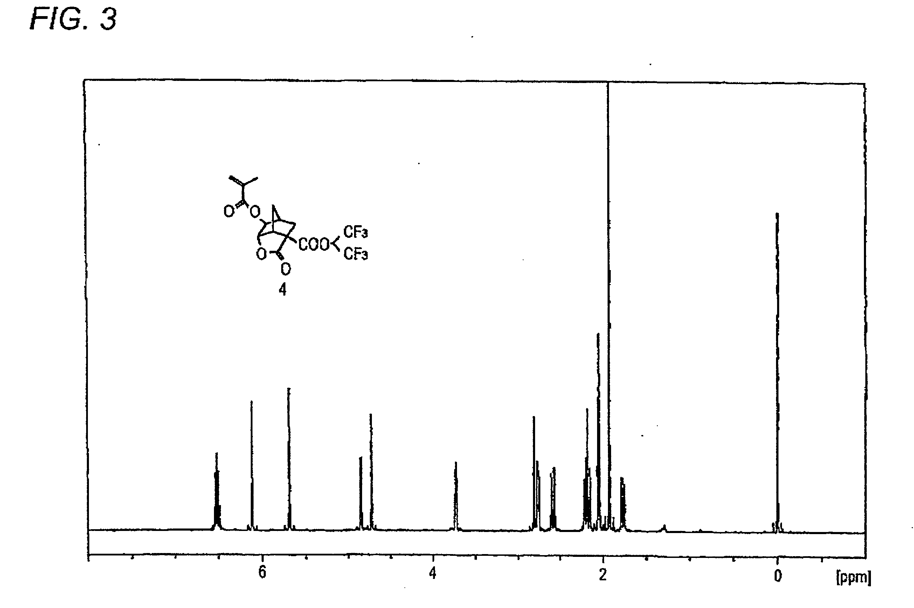

To 15.00 g of Compound (3), 300.00 g of toluene was added; 3.70 g of methacrylic acid and 4.20 g of p-toluenesulfonic acid monohydrate were further added; and the mixture was refluxed for 15 hours while removing generated water by means of azeotrope. The reaction solution was concentrated, and the concentrate was purified by means of column chromatography to obtain 11.70 g of Compound (4) shown below (yield: 65 %). A ¹H-NMR chart of Compound (4) is shown in Fig. 3,

¹H-NMR (400 MHz in (CD₃)₂CO): σ (ppm) = 1.76 to 1.79 (1H), 1.93 (3H), 2:16 to 2.22 (2H). 2.57 to 2.61 (1H), 2.76 to 2.81 (1H). 3.73 to 3.74 (1H), 4.73 (1H), 4.84 to 4.86 (1H), 5.69 to 5.70 (1H), 6.12 (1H), 6.50 to 6.56 (1H)

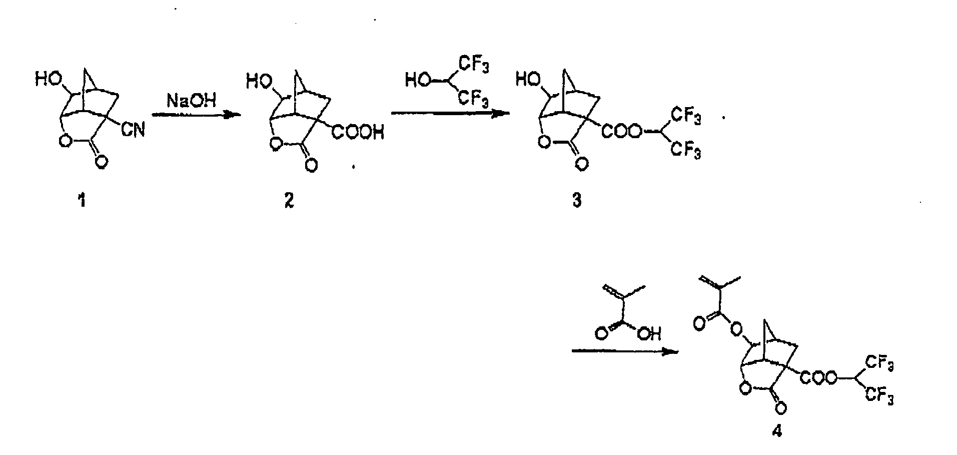

Also, Compound (4) could also be synthesized by a method shown in the following Example 1a.

### Example 1a (Synthesis of Compound (4))

A reaction was carried out under circulation with a nitrogen gas.

To 50.87 g of Compound (2), 300 g of ethyl acetate was added, and 51.76 g of 1,1,1,3,3,3-hexafluoroisopropyl alcohol and 3.18 g of 4-dimethylaminopyridine were further added, followed by stirring. 54.20 g of 1-ethyl-3-(3-dimethylaminopropyl)carbodiimide hydrochloride was added to the resulting solution; and the mixture was stirred for 5 hours. The reaction solution was added to 200 mL of 1 N HCl, thereby stopping the reaction. An organic layer was further washed with 1 N HCl and subsequently washed with water, and the organic layer was concentrated. The concentrate was subjected to azeotropic dehydration of water using volume, thereby obtaining 67.60 g of Compound (3) (yield: 76 %).

15.00 g of Compound (3) was dissolved in 67.5 g of acetonitrile. (which had been subjected to a deaeration treatment), and the solution was subjected to bubbling with a nitrogen gas. Thereafter, the resulting solution was cooled to not higher than 10°C, 8.11 g of methacrylic acid chloride was added, and 7.85 g of triethylamine was then added dropwise (the liquid temperature was kept at not higher than 10°C). Furthermore, the mixture was stirred at room temperature for 2 hours. After completion of the reaction, the reaction solution was added to 9.0 g of concentrated hydrochloric acid which had been diluted with 675 g of water and cooled to 5°C. After stirring for 30 minutes, a deposited precipitate was collected by filtration and washed with water. The resulting powder was dissolved in 45.6 g of acetonitrile, and the solution was added dropwise to 304.0 g of water at 5°C. After stirring for 30 minutes, a deposited precipitate was collected by filtration and washed with water. To the resulting powder, 76.1 g of heptane was added, and the mixture was stirred at room temperature for one hour, followed by collection by filtration and drying to obtain 13.7 g of Compound (4) (yield: 77 %).

### Example 2 (Synthesis of Compound (8)

To 17.09 g of Compound (5) shown below (methyl glycolate, manufactured by TCI), 30.00 g of tetrahydrofuran (THF) was added, and 21,15 g of triethylamine was further added. The mixture was cooled to 0°C, and 20.85 g of methacrylic acid chloride was then added dropwise. After returning the temperature to room temperature, the mixture was stirred for 2 hours. A sodium hydrogencarbonate aqueous solution was added, followed by extraction with ethyl acetate. Organic layers were gathered, to which was then added MgSO₄, and the resulting mixture was filtered and concentrated to obtain 28.51 g of Compound (6) (yield: 95 %).

1H-NMR (400 MHz in (CD₃)₂CO): σ (ppm) = 1.94 to 2.04 (3H), 3.71 to 3.72 (3H), 4.73 (2H), 5.72 (1H), 6.15 (1H)

To 28.5 g of Compound shown below (6), 180 mL of acetone was added, the mixture was cooled to 0°C, and 180 mL of a 1 N of aqueous sodium hydroxide was then added dropwise. After stirring for 30 minutes, the mixture was made acidic by the addition of hydrochloric acid and then extracted with ethyl acetate, Organic layers were gathered, to which was then added MgSO₄, and the resulting mixture was filtered and concentrated to obtain 21.2 g of Compound (7) shown below (yield: 82 %).

¹H-NMR (400 MHz in (CD₃)₂CO); σ (ppm) = 1.94 to 1.97 (3H), 4.71 to 4.72 (2H), 5.70 to 3.71 (1H), 6.15 (1H)

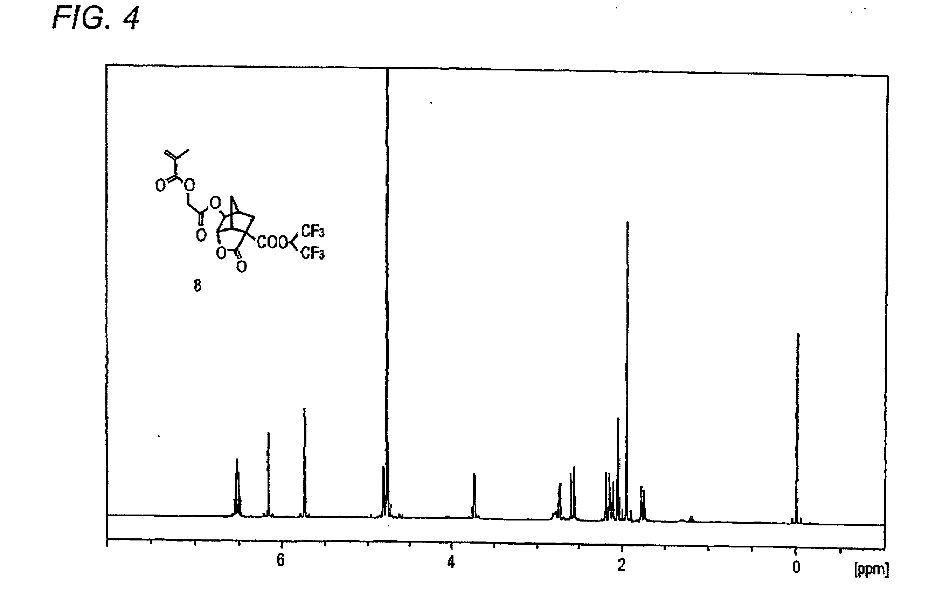

To 15.00 g of Compound (7), 300 g of toluene was added; 7.00 g of Compound (3) and 3.80 g of p-toluenesulfonic acid monohydrate were further added; and the mixture was refluxed for 6 hours while removing generated water by means of azeotrope. The reaction solution was concentrated, and the concentrate was purified by means of column chromatography to obtain 13.52 g of Compound (8) shown below (yield: 71 %). A ¹H-NMR chart of Compound (8) is shown in Fig. 4.

¹H-NMR (400 MHz in (CD₃)₂CO): σ (ppm) = 1.77 to 1.78 (1H), 1.95 to 1.96 (3H), 2.11 to 2.20 (2H), 2.56 to 2.61 (1H), 2.73 to 2.74 (1H), 3.73 to 3.75 (1H), 4.77 to 4.82 (4H), 5.74 (1H), 6.16 (1H), 6.52 to 6.53 (1H)

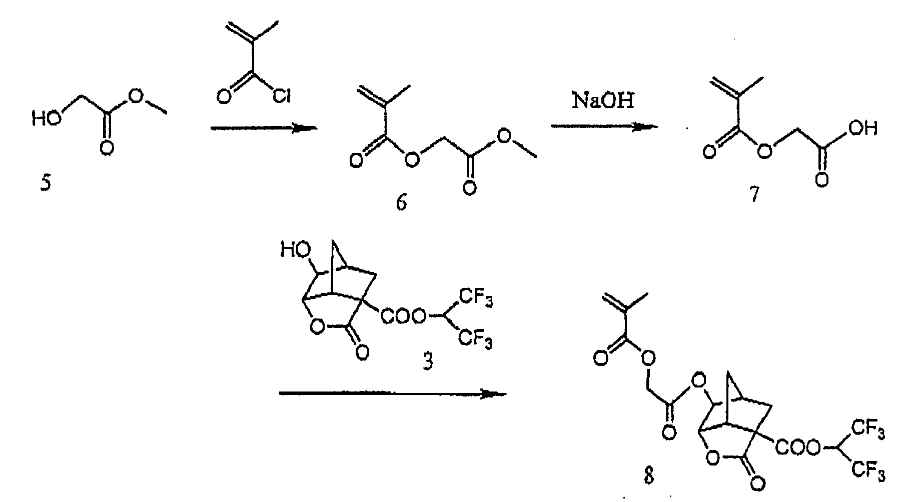

### Example 3 (Synthesis of Compound (9))

Compound (9) shown below was synthesized in the same manner as in Example 1, except for using 2,2,3,3,4,4,4-heptafluoro-1-butanol in place of the 1,1,1,3,3,3-hexafluoroisopropyl alcohol (yield of the three steps: 30 %). A ¹H-NMR chart of Compound (9) is shown in Fig, 5.

¹H-NMR (400 MHz in (CD₃)₂CO): σ (ppm) = 1.73 to 1.76 (1H), 1.93 (3H), 2.13 to 2.17 (1H), 2.57 to 2.61 (1H), 2.71 to 2.72 (1H), 2.77 to 2.81 (1H), 3.65 to 3.67 (1H), 4.69 (1H), 4.79 to 4.80 (1H), 4.91 to 5.00 (2H), 5.68 to 5.69 (1H), 6.11 to 6.12 (1H)

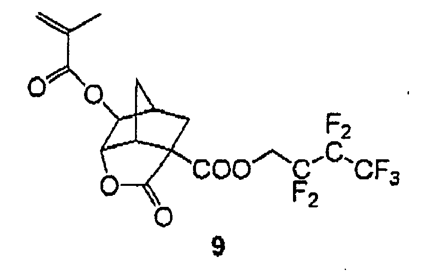

### Example 4 (Synthesis of Compound (10))

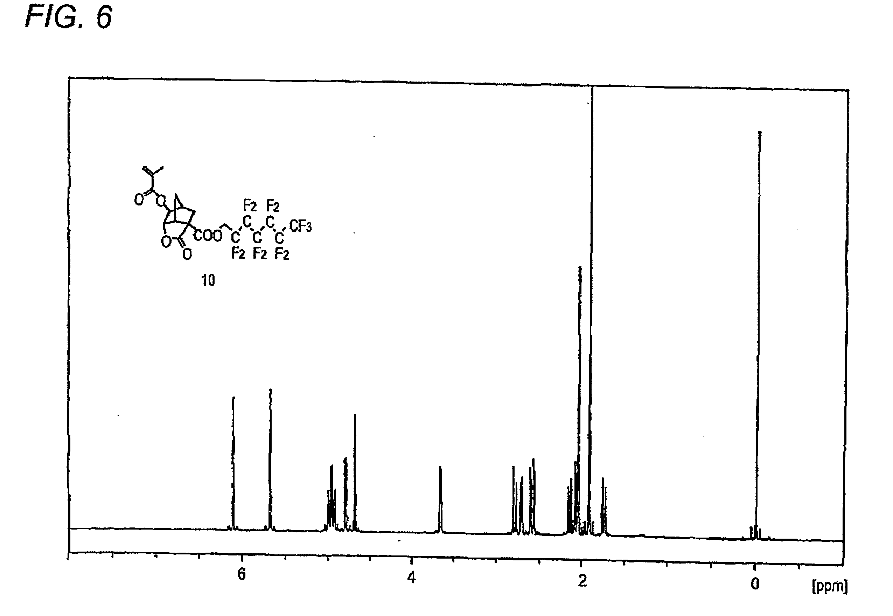

Compound (10) shown below was synthesized in the same manner as in Example 1, except for using 1H,1H-tridecafluoro-1-heptanol in place of the 1,1,1,3,3,3-hexafluoroisopropyl alcohol (yield of the three steps: 42 %). A ¹H-NMR chart of Compound (10) is shown in Fig. 6.

¹H-NMR (400 MHz in (CD₃)₂CO): σ (ppm) = 1.73 to 1.88 (1H), 1.93 (3H), 2.08 to 2.17 (1H), 2.56 to 2.61 (1H), 2.71 to 2.72 (1H), 2,77 to 2.81 (1H), 3.66 to 3.68 (1H), 4.69 (1H), 4.79 to 4.81 (1H), 4.93 to 5.01 (2H), 5.68 to 5.69 (1H), 6.11 to 6.12 (1H)

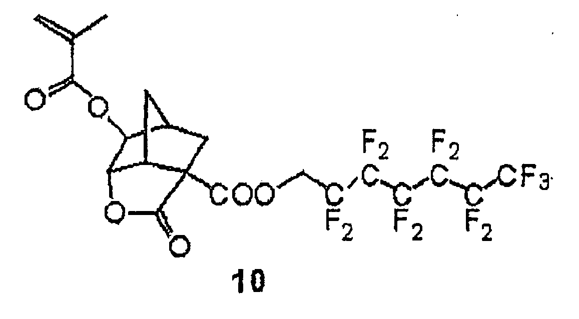

### Example 5 (Synthesis of Compound (11))

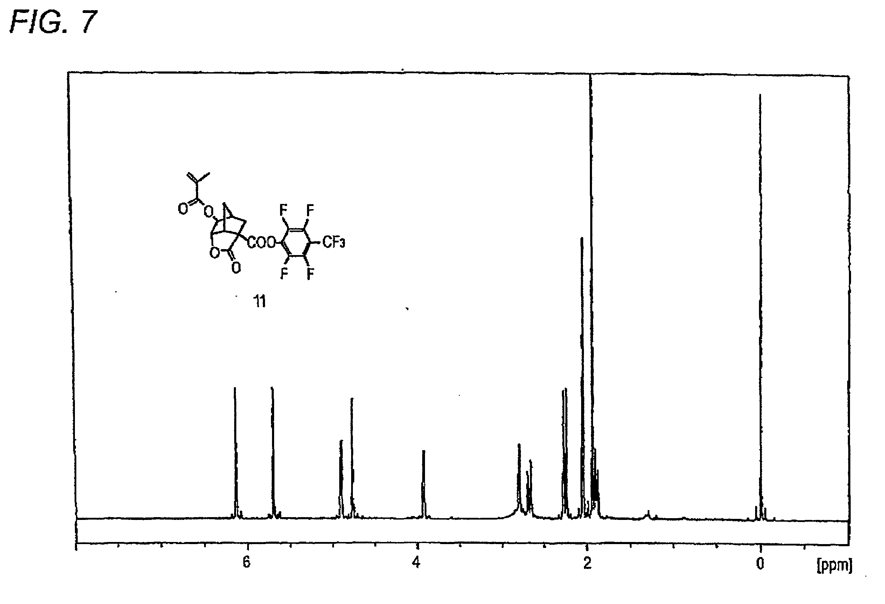

Compound (11) shown below was synthesized in the same manner as in Example 1, except for using 2,3,5,6-tetrafluoro-4-(trifluoromethyl)phenol in place of the 1,1,1,3,3,3-hexafluoroisopropyl alcohol (yield of the three steps: 23 %). A ¹H-NMR chart of Compound (11) is shown in Fig. 7.

¹H-NMR (400 MHz in (CD₃)₂CO): σ (ppm) = 1.88 to 1.91 (1H), 1.94 (3H), 2.10 to 2.28 (2H), 2.66 to 2.71 (1H), 2.80 (1H), 3.92 to 3.93 (1H), 4.77 (1H), 4.90 to 4.92 (1H), 5.70 to 5.71 (1H), 6.13 to 6.14 (1H)

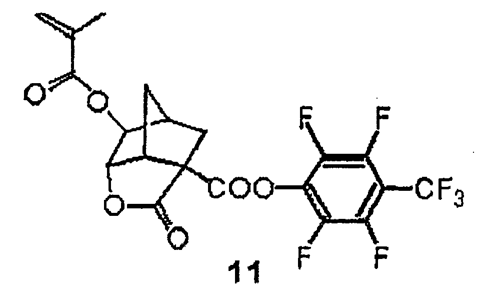

### Example 6 (Synthesis of Compound (19))

To 33,0 g of the Compound (12) shown below, 100.0 g of the Compound (13) shown below and 13.3 g of aluminum chloride, 50 g of CHCl₃ was added, and the mixture was heated at 62 °C and stirred for 10 hours. The reaction solution was added to ice water and extracted with ethyl acetate. An organic layer was concentrated, and the concentrate was then purified by means of column chromatography to obtain 13.3 g of the Compound (14) shown below (yield: 10 %).

10 g of the Compound (14) was dissolved in 150 g of CH₂Cl₂, and 10.0 g of m-chloroperbenzoic acid was gradually added to the solution while cooling at not higher than 5 °C. After 4 hours, a sodium sulfite aqueous solution was added thereto, thereby decomposing the excessive peroxide, and an organic layer was then washed with a sodium hydrogencarbonate aqueous solution, thereby obtaining an organic layer containing the Compound (15) shown below. 15 g of formic acid and 45 g of water were added to this organic layer, and the mixture was heated at 50 °C and stirred for 4 hours, After separating the organic layer, an aqueous layer was extracted with ethyl acetate. An organic layer was concentrated to obtain 9.0 g of the Compound (16) shown below (yield: 95 %).

To 10.0 g of the Compound (16), 20.0 g of water was added, and a solution of 4.7 g of NaOH dissolved in 30 g of water was further added thereto while cooling at not higher than 20 °C. The mixture was stirred for 9 hours under a heating and reflux condition. The reaction mixture was made acidic by the addition of hydrochloric acid and then extracted with ethyl acetate. An organic layer was concentrated to obtain 8.0 g of the Compound (17) shown below (yield: 90 %).

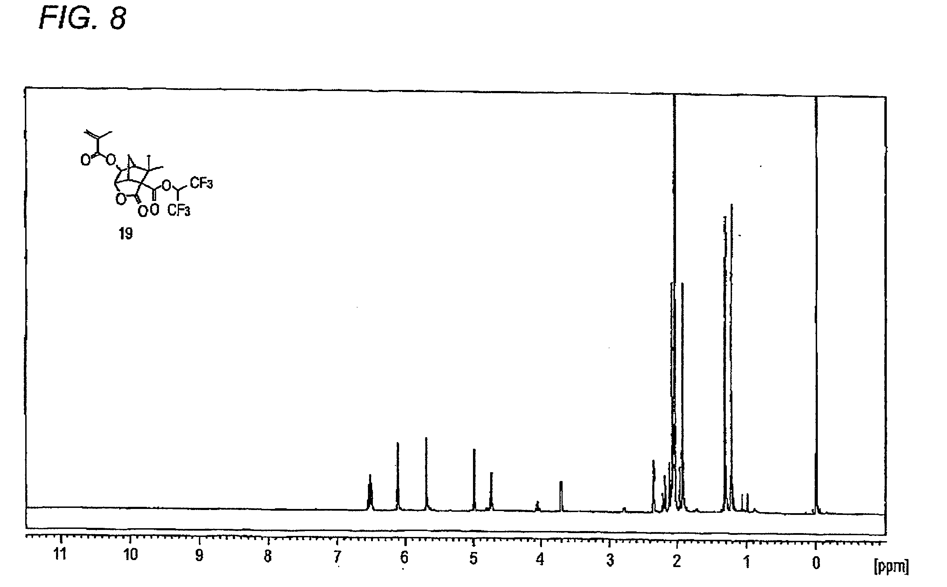

The Compound (19) shown below was synthesized via the Compound (18) shown below in the same manner as in Example 1a, except for using the Compound (17) in place of the Compound (2) (yield in the two steps: 72 %). A ¹H-NMR chart of Compound (19) is shown in Fig. 8.

¹H-NMR (400 MHz in (CD₃)₂CO): σ (ppm) = 1.23 (3H), 1.32 (3H), 1.93 (3H), 2.10 to 2.20 (2H), 2.36 (1H), 3.71 to 3.72 (1H), 4.74 to 4.75 (1H), 4.50 (1H), 5.69 to 5.70 (1H), 6.12 (1H), 6.51 to 6.54 (1H)

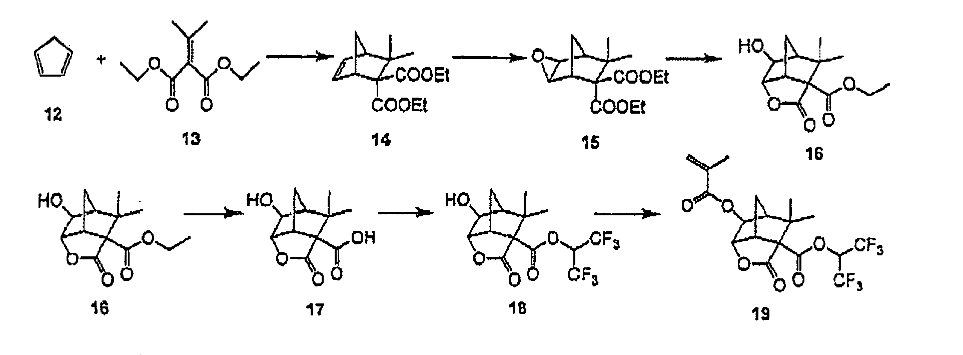

### Example 7 (Synthesis of Compound 22))

To 15.00 g of the Compound (20), 0.10 g of dimethylformamide and 25.83 g of thionyl chloride were added, and the mixture was heated at 75 °C and stirred for 2 hours. The unreacted thionyl chloride was removed under reduced pressure, thereby the Compound (21) was obtained.

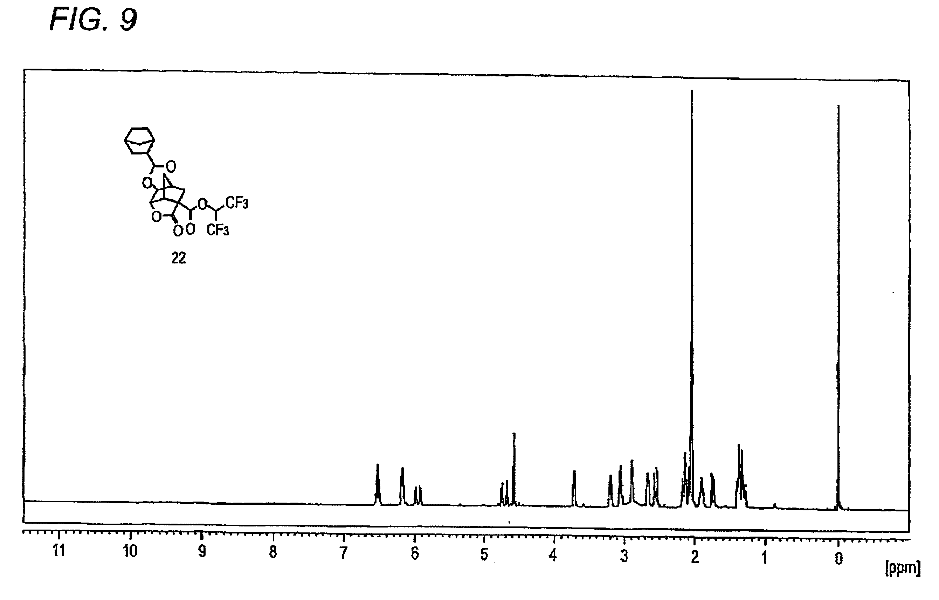

30.24 g of the Compound (3) was dissolved in 170 g of THF, and 25.76 g of pyridine and 1.33 g of 4-dimethylaminopyridine were added and the solution was stirred. The Compound (21) was dropped into the reaction solution while keeping the temperature of 5 °C or less. The reaction solution was heated at 40 °C and stirred for 9 hours. The reaction solution was dropped into the mixture solution of 600 g of IN hydrochloric acid aqueous solution and 600 g of ethyl acetate while keeping the temperature of 10 °C or less. The organic layer was washed with sodium bicarbonate aqueous solution and water and then concentrated. The concentrate was purified by means of column chromatography to obtain 16.69 g of Compound (22) (yield in the two steps: 41 %). A ¹H-NMR chart of Compound (22) is shown in Fig. 9.
¹H-NMR (400 MHz in (CD₃)₂CO): σ (ppm) = 1.29 to 1.50 (4H), 1.66 to 1.82 (1H), 1.73 to 1.82 (1H), 2.08 to 2.23 (2H), 2.53 to 2.59 (1H), 2.67 to 2.72 (1H), 3.04 to 3.08 (1H), 3.20 to 3.25 (1H), 3.68 to 3.77 (1H), 4.53 to 4.58 (1H), 4.63 to 5.36 (1H), 5.92 to 6.00 (1H), 6.13 to 6.19 (1H), 6.46 to 6.57 (1H)

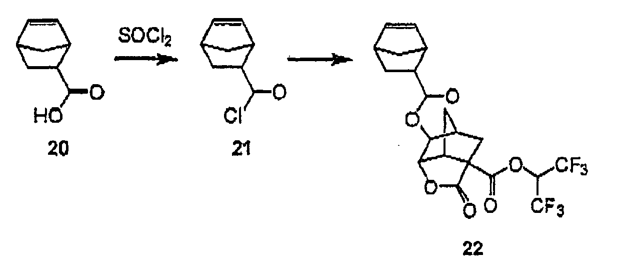

### Example 8 (Synthesis of Polymer (1))

A three-necked flask was charged with 14.03 g of PGMEA in a nitrogen atmosphere and heated at 80°C. A solution having Compound (11) (12.06 g), Compound (23) shown below (4.21 g), Compound (24) shown below (0.92 g) and 0.69 g of a polymerization initiator V-601 (manufactured by Wako Pure Chemical Industries, Ltd.) dissolved in 26.06 g of PGMEA was added dropwise thereto over 6 hours. After completion of the dropwise addition, the mixture was allowed to react at 80°C for an additional 2 hours. The reaction solution was allowed to stand for cooling and then added dropwise to a mixed solution of 620 g of methanol and 70 g of distilled water; and a deposited powder was collected by filtration and dried to obtain Polymer (1) shown below (10.01 g). The resulting polymer had a weight average molecular of 8,500 by GPC method as reduced into standard polystyrene and a degree of dispersion (Mw/Mn) of 1.51.

The NMR measurement revealed that a molar ratio of respective repeating units shown by the following structures in Polymer (1) was 50/40/10 in the order from the left-hand side repeating unit.

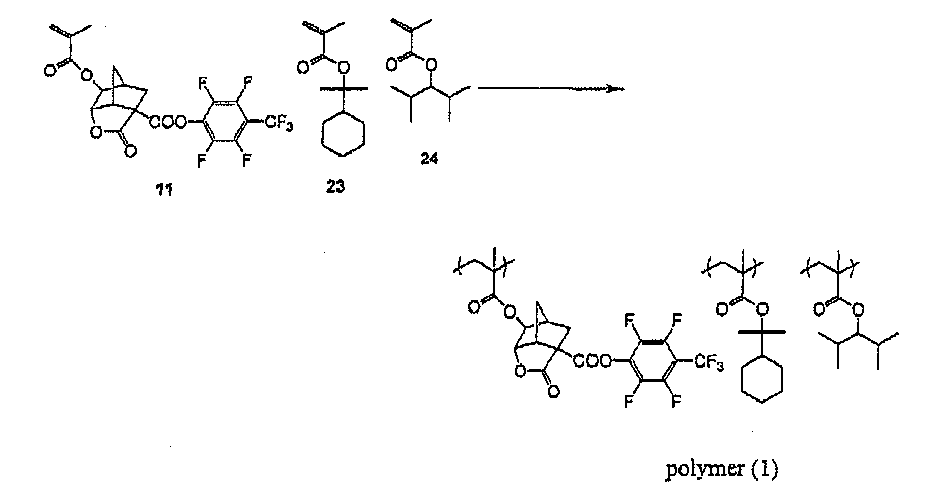

### Example 9 (Synthesis of Polymer (2) by means of batch polymerization)

A three-necked flask was charged with 19.49 g of PGMEA in a nitrogen atmosphere and heated at 80°C. A solution having Compound (10) (14.96 g), Compound (25) shown below (6.41 g), Compound (26) shown below (2.50 g) and 0.69 g of a polymerization initiator V-601 (manufactured by Wako Pure Chemical Industries, Ltd.) dissolved in 36.20 g of PGMEA was added dropwise thereto over 6 hours. After completion of the dropwise addition, the mixture was allowed to react at 80°C for an additional 2 hours. The reaction solution was allowed to stand for cooling and then added dropwise to a mixed solution of 860 g of methanol and 100 g of distilled water; and a deposited powder was collected by filtration and dried to obtain Polymer (2) shown below (13.25 g). The resulting polymer had a weight average molecular of 9,100 as reduced into standard polystyrene and a degree of dispersion (Mw/Mn) of 1.55 by GPC method.

The NMR measurement revealed that a molar ratio of respective repeating units shown by the following structures in Polymer (2) was 50/40/10 in the order from the left-hand side repeating unit.

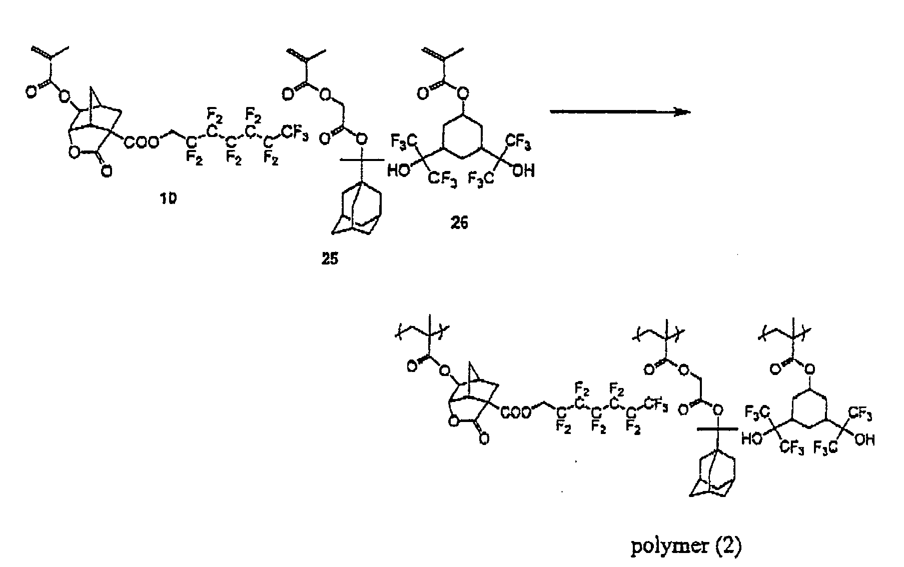

### Example 10 (Evaluation)

10 g of Polymer (1), 0.03 g of N,N-dibutylaniline, 0.30 g of triphenylsulfonium hexafluorosulfonate and 0.01 g of MEGAFAC F176 (manufactured by DIC Corporation) were dissolved in a mixed solvent of propylene glycol monomethyl ether acetate and propylene glycol monomethyl ether (mass ratio: 6/4), thereby preparing a solution having a solids concentration of 8 % by mass, which was then filtered through a polyethylene filter having a pore size of 0.03 µm. There was thus prepared a positive working resist solution.

ARC29A, manufactured by Brewer Science, Inc, was uniformly coated in a thickness of 78 nm on a silicon wafer by a spin coater and then heated for drying at 205°C for 60 seconds, thereby forming an antireflection film. Thereafter, each of the positive working resist solutions immediately after the preparation was coated by a spin coater and then dried (Pre Bake) at 115°C for 90 seconds, thereby forming a resist film having a thickness of 170 nm. This resist film was exposed through a mask by an ArF excimer laser stepper (PAS 5500/1100, manufactured by ASML, NA = 0.75 (2/3 zonal illumination)), and immediately after the exposure, the resist film was heated (Post Exposure Bake) on a hot plate at 120°C for 90 seconds. Furthermore, the resulting resist film was developed with a 2.38 % by mass tetramethylammonium hydroxide aqueous solution at 23°C for 60 seconds and then rinsed with pure water for 30 seconds. As a result, the development was achieved well, and a clear line-and-space pattern was obtained in a high precision.

### Example 11 (Evaluation)

A positive working resist solution was prepared and evaluated in the same manner as in Example 10, except for replacing 10 g of Polymer (1) with 10 g of Polymer (2). As a result, the development was achieved well, and a clear line-and-space pattern was obtained in a high precision.

### Examples 12 and 13 (Evaluation)

The same operations as in Examples 10 and 11 were carried out, respectively, except that in Examples 10 and 11, the ArF excimer laser stepper was replaced by an ArF excimer laser immersion scanner (XT1700i, manufactured by ASML, No = 1.20, C-Quad, outer sigma: 0.981, inner sigma: 0.895, xy-polarization) (immersion liquid; ultra pure water). As a result, the development was achieved well, and a clear line-and-space pattern was obtained in a high precision. According to the invention, it is possible to provide a novel polymerizable compound, a lactone-containing compound, a method for manufacturing a lactone-coutaining compound and a polymer compound obtained by polymerizing the instant polymerizable compound. The polymer compound of the invention is, for example, useful for the formation of a fine pattern in the semiconductor manufacture

Especially, the novel polymer compound which is obtained from the novel polymerizable compound of the invention is useful as a polymer compound to be added to a resist composition which is used for the formation of a fine pattern in the semiconductor manufacture, such as a resist composition suitable for exposure by a liquid immersion type projection exposure system, and is able to provide a resist composition which is excellent in an affinity with a developing solution, in particular, alkaline developability.

## Claims

1. A polymerizable compound having a lactone structure, the polymerizable compound being represented by the following formula (1): 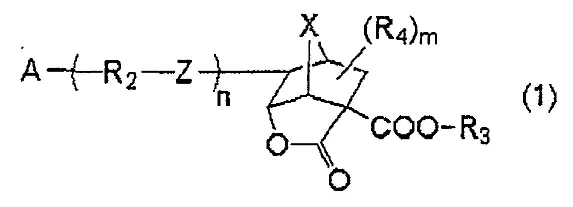 wherein
A represents a polymerizable site;
R₂ represents a single bond or a chain or cyclic alkylene group which may have a substituent, and when plural R₂s are present, R₂s may be the same as or different from every other R₂;
R₃ represents a linear, branched or cyclic hydrocarbon group in which a part or all of hydrogen atoms linked to a constituent carbon atom or carbon atoms are substituted with a fluorine atom and which may further have a substituent;
R₄ represents a halogen atom, a cyano group, a hydroxy group, an amide group, an alkyl group which may have a substituent, a cycloalkyl group which may have a substituent, an alkoxy group which may have a substituent, a phenyl group which may have a substituent, an acyl group which may have a substituent, an alkoxycarbonyl group which may have a substituent, or a group represented by R-C(=O)- or R-C(=O)O-, wherein R represents an alkyl group which may have a substituent or a cycloalkyl group which may have a substituent; and when plural R₄s are present, R₄s may be the same as or different from every other R₄, and two or more R₄s may be bonded to each other to form a ring;
X represents an alkylene group which may have a substituent, an oxygen atom or a sulfur atom;
Z represents a single bond, an ether bond, an ester bond, an amide bond, a urethane bond or a urea bond, and when plural Zs are present, Zs may be the same as or different from every other Z;
n represents a repetition number and represents an integer of front 0 to 5; and
m represents the number of substituents and represents an integer of from 0 to 7.

2. The polymerizable compound according to claim 1, which is represented by the following formula (2): 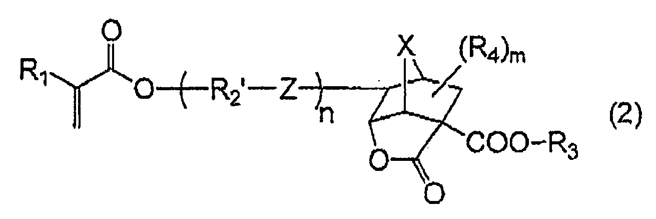 wherein
R₁ represents a hydrogen atom, an alkyl group which may have a substituent, or a halogen atom;
R₂' represents a chain or cyclic alkylene group which may have a substituent, and when plural R'₂s are present, R'₂s may be the same as or different from every other R'₂;
R₃ represents a linear, branched or cyclic hydrocarbon group in which a part or all of hydrogen atoms linked to a constituent carbon atom or carbon atoms are substituted with a fluorine atom and which may further have a substituent;
R₄ represents a halogen atom, a cyano group, a hydroxy group, an amide group, an alkyl group which may have a substituent, a cycloalkyl group which may have a substituents, an alkoxy group which may have a substituent, a phenyl group which may have a substituent, an acyl group which may have a substituent, an alkoxycarbonyl group which may have a substituent, or a group represented by R-C(=O)- or R-C(=O)O-, wherein R represents an alkyl group which may have a substituent or a cycloalkyl group which may have a substituent; and when plural R₄s are present, R₄s may be the same as or different from every other R₄, and two or more R₄s may be bonded to each other to form a ring;
X represents an alkylene group which may have a substituent, an oxygen atom or a sulfur atom;
Z represents a single bond, an ether bond, an ester bond, an amide bond, a urethane bond or a urea bond, and when plural Zs are present, Zs may be the same as or different from every other Z;
n represents a repetition number and represents an integer of from 0 to 5; and
m represents the number of substituents and represents an integer of from 0 to 7.

3. The polymerizable compound according to claim 1, which is represented by the following formula (3): 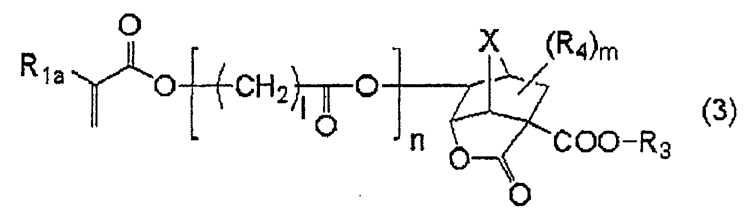 wherein
R₁ₐ represents a hydrogen atom, a methyl group, a hydroxymethyl group, a halogenated methyl group or a halogen atom;
R₃ represents a linear, branched or cyclic hydrocarbon group in which a part or all of hydrogen atoms linked to a constituent carbon atom or carbon atoms are substituted with a fluorine atom and which may further have a substituent;
R₄ represents a halogen atom, a cyano group, a hydroxy group, an amide group, an alkyl group which may have a substituent, a cycloalkyl group which may have a substituent, an alkoxy group which may have a substituent, a phenyl group which may have a substituent, an acyl group which may have a substituent, an alkoxycarbonyl group which may have a substituent or a group represented by R-C(=O)- or R-C(=O)O-, wherein R represents an alkyl group which may have a substituent or a cycloalkyl group which may have a substituent; and when plural R₄s are present, R₄s may be the same as or different from every other R₄, and two or more R₄s may be bonded to each other to form a ring;
X represents an alkylene group which may have a substituent, an oxygen atom or a sulfur atom;
I represents a repetition number and represents an integer of from 1 to 5;
n represents a repetition number and represents an integer of from 0 to 5; and
m represents the number of substituents and represents an integer of from 0 to 7.

4. The polymerizable compound according to claim 1,
wherein, in the formula (1), R₃ represents a linear hydrocarbon group in which a part or all of hydrogen atoms linked to a constituent carbon atom or carbon atoms are substituted with a fluorine atom, and
the .linear hydrocarbon group is a group represented by -(CH₂)ₙ-(CF₂)ₘ-CF₃ where n represents an integer of 0 or 1 and m represents an integer of from 0 to 10.

5. The polymerizable compound according to claim 1,
wherein, in the formula (1), R₃ represents a branched hydrocarbon group in which a part or all of hydrogen atoms linked to a constituent carbon atom or carbon atoms are substituted with a fluorine atom, and
the branched hydrocarbon group is a group represented by -C(Ra)(Rb)(Rc), where Ra, Rb and Rc each independently represents a hydrogen atom, an alkyl group, a cycloalkyl group or an aryl group provided that at least one of Ra, Rb and Rc represents an alkyl group having a fluorine atom as a substituent, a cycloalkyl group having a fluorine atom as a substituent or an aryl group having a fluorine atom as a substituent.

6. The polymerizable compound according to claim 1,
wherein, in the formula (1), R₃ represents a cyclic hydrocarbon group in which a part or all of hydrogen atoms linked to a constituent carbon atom or carbon atoms are substituted with a fluorine atom, and
the cyclic hydrocarbon group is a phenyl group having a fluorine atom as a substituent and which may further have a substituent, a cyclopentyl group having a fluorine atom as a substituent and which may further have a substituent or a cyclohexyl group having a fluorine atom as a substituent and which may further have a substituent.

7. A lactone-containing compound represented by the following formula (4): 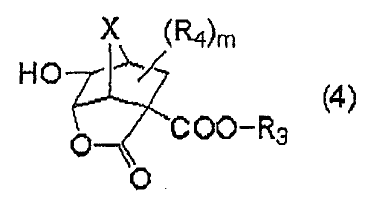 wherein
R₃ represents a linear, branched or cyclic hydrocarbon group in which a part or all of hydrogen atoms linked to a constituent carbon atom or carbon atoms are substituted with a fluorine atom and which may further have a substituent;
R₄ represents a halogen atom, a cyano group, a hydroxy group, an amide group, an alkyl group which may have a substituent, a cycloalkyl group which may have a substituent, an alkoxy group which may have a substituent, a phenyl group which may have a substituent, an acyl group which may have a substituents, an alkoxycarbonyl group which may have a substituent or a group represented by R-C(=O)- or R-C(=O)O-, wherein R represents an alkyl group which may have a substituent or a cycloalkyl group which may have a substituent; and when plural R₄s are present, R₄s may be the same as or different from every other R₄, and two or more R₄s may be bonded to each other to form a ring;
X represents an alkylene group which may have a substituent, an oxygen atom or a sulfur atom; and
m represents the number of substituents and represents an integer of from 0 to 7.

8. A method for manufacturing a lactone-containing compound represented by the following formula (4), the method comprising:
reacting a carboxylic acid-containing compound represented by the following formula (5) with an alcohol represented by the following formula (6): 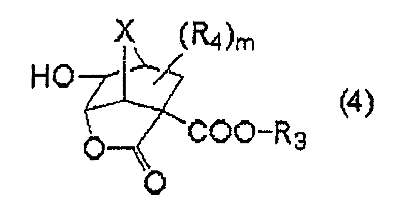 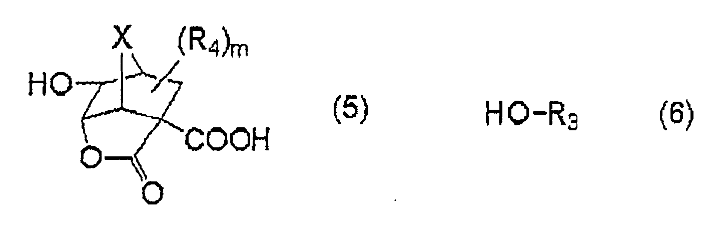 wherein
R₃ represents a linear, branched or cyclic hydrocarbon group in which a part or all of hydrogen atoms linked to a constituent carbon atom or carbon atoms are substituted with a fluorine atom and which may further have a substituent;
R₄ represents a halogen atom, a cyano group, a hydroxy group, an amide group, an alkyl group which may have a substituent, a cycloalkyl group which may have a substituent, an alkoxy group which may have a substituent, a phenyl group which may have a substituent, an acyl group which may have a substituent, an alkoxycarbonyl group which may have a substituent or a group represented by R-C(=O)- or R-C(=O)O-, wherein R represents an alkyl group which may have a substituent or a cycloalkyl group which may have a substituent; and when plural R₄s are present, R₄s may be the same as or different from every other R₄, and two or more R₄s may be bonded to each other to form a ring;
X represents an alkylene group which may have a substituent, an oxygen atom or a sulfur atom; and
m represents the number of substituents and represents an integer of from 0 to 7.

9. A polymer compound obtained by polymerizing the polymerizable compound according to claim 1.

## Patentansprüche

1. Polymerisierbare Verbindung mit einer Lactonstruktur, wobei die polymerisierbare Verbindung durch die folgende Formel (1) dargestellt ist: 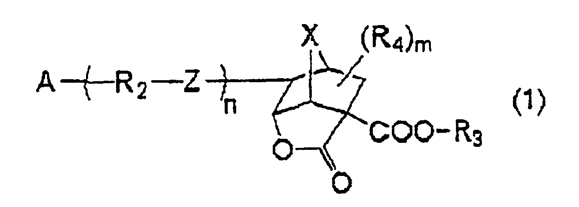 worin
A eine polymerisierbare Stelle ist;
R₂ eine Einfachbindung oder eine kettenförmige oder cyclische Alkylengruppe ist, die einen Substituenten aufweisen kann und wenn mehrere R₂s vorliegen, können die R₂s gleich oder verschieden von anderen R₂s sein;
R₃ eine lineare, verzweigte oder cyclische Kohlenwasserstoffgruppe ist, in der ein Teil oder alle der Wasserstoffatome, die mit einem konstituierenden Kohlenstoffatom oder Kohlenstoffatomen verknüpft sind, mit einem Fluoratom substituiert sind und die weiterhin einen Substituenten aufweisen können;
R₄ ein Halogenatom, eine Cyanogruppe, eine Hydroxygruppe, eine Amidgruppe, eine Alkylgruppe, die einen Substituenten aufweisen kann, eine Cycloalkylgruppe, die einen Substituenten aufweisen kann, eine Alkoxygruppe, die einen Substituenten aufweisen kann, eine Phenylgruppe, die einen Substituenten aufweisen kann, eine Acylgruppe, die einen Substituenten aufweisen kann, eine Alkoxycarbonylgruppe, die einen Substituenten aufweisen kann oder eine Gruppe ist, die durch R-C(=O)- oder R-C(=O)O- dargestellt ist, worin R eine Alkylgruppe, die einen Substituenten aufweisen kann oder eine Cycloalkylgruppe, die einen Substituenten aufweisen, ist und wenn mehrere R₄s vorliegen, können die R₄s gleich oder verschieden von anderen R₄s sein und zwei oder mehrere R₄s können zur Bildung eines Rings miteinander verbunden sein;
X eine Alkylengruppe, die einen Substituenten aufweisen kann, ein Sauerstoffatom oder ein Schwefelatom ist;
Z eine Einfachbindung, eine Etherbindung, eine Esterbindung, eine Amidbindung, eine Urethanbindung oder eine Ureabindung ist und wenn mehrere Zs vorliegen, können die Zs gleich oder verschiedenen von anderen Zs sein;
n eine Wiederholungsanzahl und eine ganze Zahl von 0 bis 5 ist; und
m die Anzahl der Substituenten und eine ganze Zahl von 0 bis 7 ist.

2. Polymerisierbare Verbindung gemäß Anspruch 1, die durch folgende Formel (2) dargestellt ist: 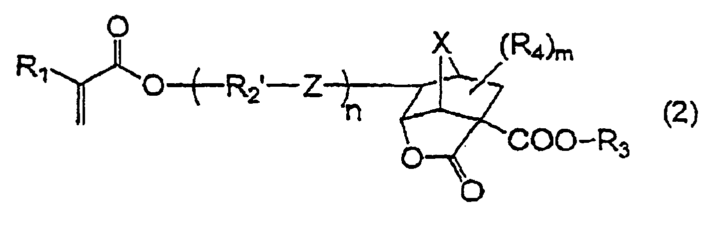 worin
R₁ ein Wasserstoffatom, eine Alkylgruppe, die einen Substituenten aufweisen kann, oder ein Halogenatom ist;
R_{2'} eine kettenförmige oder cyclische Alkylengruppe ist, die einen Substituenten aufweisen kann und wenn mehrere R₂s vorliegen, können die R₂s gleich oder verschieden von anderen R₂s sein;
R₃ eine lineare, verzweigte oder cyclische Kohlenwasserstoffgruppe ist, in der ein Teil oder alle der Wasserstoffatome, die an ein konstituierendes Kohlenstoffatom oder an Kohlenstoffatome verknüpft sind, durch ein Fluoratom substituiert sind und die weiterhin einen Substituenten aufweisen können;
R₄ ein Halogenatom, eine Cyanogruppe, eine Hydroxygruppe, eine Amidgruppe, eine Alkylgruppe, die einen Substituenten aufweisen kann, eine Cycloalkylgruppe, die einen Substituenten aufweisen kann, eine Alkoxygruppe, die einen Substituenten aufweisen kann, eine Phenylgruppe, die einen Substituenten aufweisen kann, eine Acylgruppe, die einen Substituenten aufweisen kann, eine Alkoxycarbonylgruppe, die einen Substituenten aufweisen kann oder eine Gruppe ist, die durch R-C(=O)- oder R-C(=O)O- dargestellt ist, worin R eine Alkylgruppe, die einen Substituenten aufweisen kann oder eine Cycloalkylgruppe, die einen Substituenten aufweisen, ist; und wenn mehrere R₄s vorliegen, können die R₄s gleich oder verschieden von anderen R₄s sein und zwei oder mehrere R₄s können zur Bildung eines Rings miteinander verbunden sein;
X eine Alkylengruppe, die einen Substituenten aufweisen kann, ein Sauerstoffatom oder ein Schwefelatom ist;
Z eine Einfachbindung, eine Etherbindung, eine Esterbindung, eine Amidbindung, eine Urethanbindung oder eine Ureabindung ist und wenn mehrere Zs vorliegen, können die Zs gleich oder verschiedenen von anderen Zs sein;
n eine Wiederholungsanzahl und eine ganze Zahl von 0 bis 5 ist; und
m die Anzahl der Substituenten und eine ganze Zahl von 0 bis 7 ist.

3. Polymerisierbare Verbindung gemäß Anspruch 1, die durch folgende Formel (3) dargestellt ist: 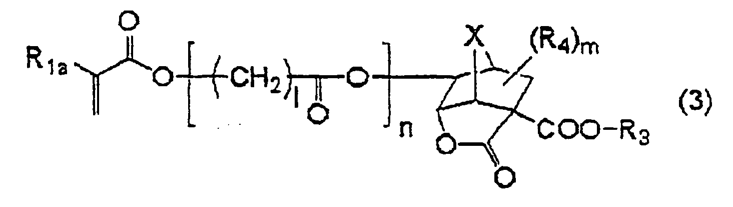 worin
R₁ₐ ein Wasserstoffatom, eine Methylgruppe, eine Hydroxymethylgruppe, eine halogenierte Methylgruppe oder ein Halogenatom ist;
R₃ eine linear, verzweigte oder cyclische Kohlenwasserstoffgruppe ist, in der ein Teil oder alle der Wasserstoffatome, die an ein konstituierendes Kohlenstoffatom oder Kohlenstoffatome verknüpft sind, mit einem Fluoratom substituiert sind und die weiterhin einen Substituenten aufweisen können;
R₄ ein Halogenatom, eine Cyanogruppe, eine Hydroxygruppe, eine Amidgruppe, eine Alkylgruppe, die einen Substituenten aufweisen kann, eine Cycloalkylgruppe, die einen Substituenten aufweisen kann, eine Alkoxygruppe, die einen Substituenten aufweisen kann, eine Phenylgruppe die einen Substituenten aufweisen kann, eine Acylgruppe, die einen Substituenten aufweisen kann, eine Alkoxycarbonylgruppe, die einen Substituenten aufweisen kann oder eine Gruppe ist, die durch R-C(=O)- oder R-C(=O)O- dargestellt ist ist, worin R eine Alkylgruppe, die einen Substituenten aufweisen kann oder eine Cycloalkylgruppe, die einen Substituenten aufweisen, ist; und wenn mehrere R₄s vorliegen, können die R₄s gleich oder verschieden von anderen R₄s sein und zwei oder mehrere R₄s können zur Bildung eines Rings miteinander verbunden sein;
X eine Alkylengruppe, die einen Substituenten aufweisen kann, ein Sauerstoffatom oder ein Schwefelatom ist;
l eine Wiederholungsanzahl und eine ganze Zahl von 1 bis 5 ist;
n eine Wiederholungsanzahl und eine ganze Zahl von 0 bis 5 ist; und
m die Anzahl der Substituenten und eine ganze Zahl von 0 bis 7 ist.

4. Polymerisierbare Verbindung gemäß Anspruch 1, worin in Formel (1) R₃ eine lineare Kohlenwasserstoffgruppe ist, in der ein Teil oder alle der Wasserstoffatome die in ein konstituierendes Kohlenstoffatom oder Kohlenstoffatome verknüpft sind mit einem Fluoratom substituiert sind und
die lineare Kohlenwasserstoffgruppe eine Gruppe ist, die durch -(CH₂)ₙ-(CF₂)ₘ-CF₃ dargestellt ist, worin n eine ganze Zahl von 0 bis 1 ist und n eine ganze Zahl von 0 bis 10 ist.

5. Polymerisierbare Verbindung gemäß Anspruch 1, worin in Formel (1) R₃ eine verzweigte Kohlenwasserstoffgruppe ist, in der ein Teil oder alle der Wasserstoffatome, die an ein konstituierendes Kohlenstoffatom oder Kohlenstoffatome gebunden sind, mit einem Fluoratom substituiert sind und
die verzweigte Kohlenwasserstoffgruppe eine Gruppe ist, die durch -C(Ra)(Rb)(Rc) dargestellt ist, worin Ra, Rb und Rc jeweils unabhängig voneinander ein Wasserstoffatom, eine Alkylgruppe, eine Cycloalkylgruppe oder eine Arylgruppe ist, vorausgesetzt dass mindestens eines der Ra, Rb und Rc eine Alkylgruppe mit einem Fluoratom als Substituenten, eine Cycloalkylgruppe mit einem Fluoratom als Substituenten oder eine Arylgruppe mit einem Fluoratom als Substituenten ist.

6. Polymerisierbare Verbindung gemäß Anspruch 1, worin in Formel (1) R₃ eine cyclische Kohlenwasserstoffgruppe ist, in der ein Teil oder alle der Kohlenstoffatome, die an ein konstituierendes Kohlenstoffatom oder Kohlenstoffatome gebunden sind, mit einem Fluoratom substituiert sind und
die cyclische Kohlenwasserstoffgruppe eine Phenylgruppe mit einem Fluoratom als Substituenten und der weiterhin einen Substituenten aufweisen kann, eine Cyclopentylgruppe mit einem Fluoratom als Substituenten und der weiterhin einen Substituenten aufweisen kann oder eine Cyclohexylgruppe mit einem Fluoratom als Substituenten und der weiterhin einen Substituenten aufweisen kann ist.

7. Lacton-haltige Verbindung der folgenden Formel (4): 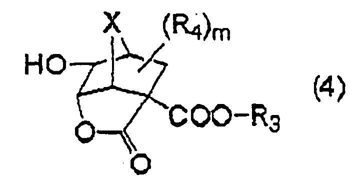 worin
R₃ eine lineare, verzweigte oder cyclische Kohlenwasserstoffgruppe ist, in der ein Teil oder alle der Wasserstoffatome, die an ein konstituierendes Kohlenstoffatom oder Kohlenstoffatome gebunden sind, mit einem Fluoratom substituiert sind und die weiterhin einen Substituenten aufweisen können;
R₄ ein Halogenatom, eine Cyanogruppe, eine Hydroxygruppe, eine Amidgruppe, eine Alkylgruppe, die einen Substituenten aufweisen kann, eine Cycloalkylgruppe, die einen Substituenten aufweisen kann, eine Alkoxygruppe, die einen Substituenten aufweisen kann, eine Phenylgruppe die einen Substituenten aufweisen kann, eine Acylgruppe, die einen Substituenten aufweisen kann, eine Alkoxycarbonylgruppe, die einen Substituenten aufweisen kann oder eine Gruppe ist, die durch R-C(=O)- oder R-C(=O)O- dargestellt ist, worin R eine Alkylgruppe, die einen Substituenten aufweisen kann oder eine Cycloalkylgruppe, die einen Substituenten aufweisen, ist; und wenn mehrere R₄s vorliegen, können die R₄s gleich oder verschieden von anderen R₄s sein und zwei oder mehr R₄s können zur Bildung eines Rings miteinander verbunden sein;
X eine Alkylengruppe, die einen Substituenten aufweisen kann, ein Sauerstoffatom oder ein Schwefelatom ist; und
m die Anzahl der Substituenten und eine ganze Zahl von 0 bis 7 ist.

8. Verfahren zur Herstellung einer Lacton-haltigen Verbindung, die durch folgende Formel (4) dargestellt ist, wobei das Verfahren folgendes umfasst:
Reagieren einer Carbonsäure-haltigen Verbindung der folgenden Formel (5) mit einem Alkohol der folgenden Formel (6): 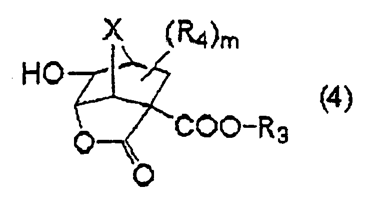 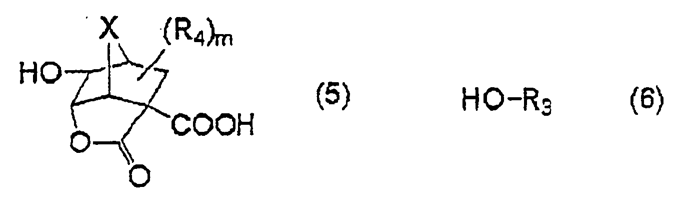 worin
R₃ eine lineare, verzweigte oder cyclische Kohlenwasserstoffgruppe ist, in der ein Teil oder alle der Wasserstoffatome, die an ein konstituierendes Kohlenstoffatom oder Kohlenstoffatome gebunden sind mit einem Fluoratom substituiert sind und die weiterhin einen Substituenten aufweisen können;
R₄ ein Halogenatom, eine Cyanogruppe, eine Hydroxygruppe, eine Amidgruppe, eine Alkylgruppe, die einen Substituenten aufweisen kann, eine Cycloalkylgruppe, die einen Substituenten aufweisen kann, eine Alkoxygruppe, die einen Substituenten aufweisen kann, eine Phenylgruppe die einen Substituenten aufweisen kann, eine Acylgruppe, die einen Substituenten aufweisen kann, eine Alkoxycarbonylgruppe, die einen Substituenten aufweisen kann oder eine Gruppe ist, die durch R-C(=O)- oder R-C(=O)O- dargestellt ist ist, worin R eine Alkylgruppe, die einen Substituenten aufweisen kann oder eine Cycloalkylgruppe, die einen Substituenten aufweisen, ist; und wenn mehrere R₄s vorliegen, können die R₄s gleich oder verschieden von anderen R₄s sein und zwei oder mehr R₄s können zur Bildung eines Rings miteinander verbunden sein;
X eine Alkylengruppe, die einen Substituenten aufweisen kann, ein Sauerstoffatom oder ein Schwefelatom ist; und
m die Anzahl der Substituenten und eine ganze Zahl von 0 bis 7 ist.

9. Polymerverbindung erhältlich durch Polymerisation der polymerisierbaren Verbindung gemäß Anspruch 1.

## Revendications

1. Composé polymérisable à structure de lactone, le composé polymérisable étant représenté par la formule (1) ci-dessous : 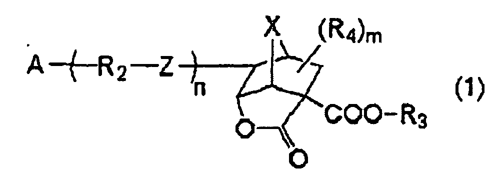 dans laquelle
A représente un site polymérisable,
R₂ représente une liaison simple ou un groupement alkylène, qui peut porter un substituant et, lorsque plusieurs R₂ sont présents, les R₂ peuvent être identiques ou différents de tout autre R₂,
R₃ représente un groupement hydrocarboné linéaire, ramifié ou cyclique, dans lequel un partie, ou tous les atomes d'hydrogène liés à un ou des atomes de carbone constituants sont substitués par un atome de fluor, et qui peut en outre porter un substituant,
R₄ représente un atome d'halogène, un groupement cyano, un groupement hydroxy, un groupement amide, un groupement alkyle qui peut porter un substituant, un groupement cycloalkyle qui peut porter un substituant, un groupement alkoxy qui peut porter un substituant, un groupement phényle qui peut porter un substituant, un groupement acyle qui peut porter un substituant, un groupement alkoxycarbonyle qui peut porter un substituant, ou un groupement représenté par R-C(=O)- ou R-C(=O)O-, où R représente un groupement alkyle qui peut porter un substituant, ou un groupement cycloalkyle qui peut porter un substituant et, lorsque plusieurs R₄ sont présents, les R₄ peuvent être identiques ou différents de tout autre R₄, et deux R₄ ou plus peuvent être liés l'un à l'autre pour former un cycle,
X représente un groupement alkylène qui peut porter un substituant, un atome d'oxygène ou un atome de soufre,
Z représente une liaison simple, une liaison éther, une liaison ester, une liaison amide, une liaison uréthane ou une liaison urée et, lorsque plusieurs Z sont présents, les Z peuvent être identiques ou différents de tout autre Z,
n représente un nombre de répétition, et est un entier de 0 à 5, et
m représente le nombre de substituants et est un entier de 0 à 7.

2. Composé polymérisable selon la revendication 1, qui est représenté par la formule (2) ci-dessous 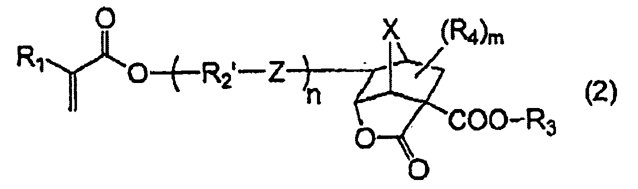 dans laquelle
R₁ représente un atome d'hydrogène, un groupement alkyle qui peut porter un substituant, ou un atome d'halogène,
R'₂ représente un groupement alkylène à chaîne ou cyclique, qui peut porter un substituant et, lorsque plusieurs R'₂ sont présents, les R'₂ peuvent être identiques ou différents de tout autre R'₂,
R₃ représente un groupement hydrocarboné linéaire, ramifié ou cyclique, dans lequel un partie, ou tous les atomes d'hydrogène liés à un ou des atomes de carbone constituants sont substitués par un atome de fluor, et qui peut en outre porter un substituant,
R₄ représente un atome d'halogène, un groupement cyano, un groupement hydroxy, un groupement amide, un groupement alkyle qui peut porter un substituant, un groupement cycloalkyle qui peut porter un substituant, un groupement alkoxy qui peut porter un substituant, un groupement phényle qui peut porter un substituant, un groupement acyle qui peut porter un substituant, un groupement alkoxycarbonyle qui peut porter un substituant, ou un groupement représenté par R-C(=O)- ou R-C(=O)O-, où R représente un groupement alkyle qui peut porter un substituant, ou un groupement cycloalkyle qui peut porter un substituant et, lorsque plusieurs R₄ sont présents, les R₄ peuvent être identiques ou différents de tout autre R₄, et deux R₄ ou plus peuvent être liés l'un à l'autre pour former un cycle,
X représente un groupement alkylène qui peut porter un substituant, un atome d'oxygène ou un atome de soufre,
Z représente une liaison simple, une liaison éther, une liaison ester, une liaison amide, une liaison uréthane ou une liaison urée et, lorsque plusieurs Z sont présents, les Z peuvent être identiques ou différents de tout autre Z,
n représente un nombre de répétition, et est un entier de 0 à 5, et
m représente le nombre de substituants et est un entier de 0 à 7.

3. Composé polymérisable selon la revendication 1, qui est représenté par la formule (3) ci-dessous 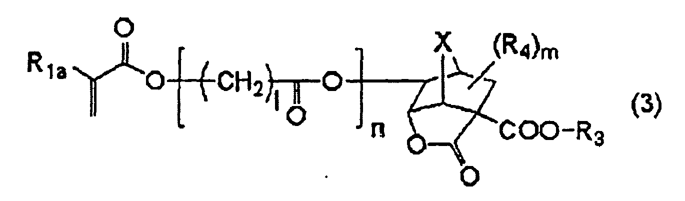 dans laquelle
R₁ₐ représente un atome d'hydrogène, un groupement méthyle, un groupement hydroxyméthyle, un groupement méthyle halogéné, ou un atome d'halogène,
R₃ représente un groupement hydrocarboné linéaire, ramifié ou cyclique, dans lequel un partie, ou tous les atomes d'hydrogène liés à un ou des atomes de carbone constituants sont substitués par un atome de fluor, et qui peut en outre porter un substituant,
R₄ représente un atome d'halogène, un groupement cyano, un groupement hydroxy, un groupement amide, un groupement alkyle qui peut porter un substituant, un groupement cycloalkyle qui peut porter un substituant, un groupement alkoxy qui peut porter un substituant, un groupement phényle qui peut porter un substituant, un groupement acyle qui peut porter un substituant, un groupement alkoxycarbonyle qui peut porter un substituant, ou un groupement représenté par R-C(=O)- ou R-C(=O)O-, où R représente un groupement alkyle qui peut porter un substituant, ou un groupement cycloalkyle qui peut porter un substituant et, lorsque plusieurs R₄ sont présents, les R₄ peuvent être identiques ou différents de tout autre R₄, et deux R₄ ou plus peuvent être liés l'un à l'autre pour former un cycle,
X représente un groupement alkylène qui peut porter un substituant, un atome d'oxygène ou un atome de soufre,
1 représente un nombre de répétition, et est un entier de 1 à 5
n représente un nombre de répétition, et est un entier de 0 à 5, et
m représente le nombre de substituants et est un entier de 0 à 7.

4. Composé polymérisable selon la revendication 1,
dans lequel, dans la formule (1), R₃ représente un groupement hydrocarboné linéaire dans lequel une partie ou tous les atomes d'hydrogène liés à un ou des atomes de carbone constituants sont substitués par un atome de fluor, et
le groupement hydrocarboné linéaire est un groupement représenté par -(CH₂)ₙ-(CF₂)ₘ-CF₃, où n est un entier valant 0 ou 1, et m représente un entier de 0 à 10.

5. Composé polymérisable selon la revendication 1,
dans lequel, dans la formule (1), R₃ représente un groupement hydrocarboné ramifié dans lequel une partie ou tous les atomes d'hydrogène liés à un ou des atomes de carbone constituants sont substitués par un atome de fluor, et
le groupement hydrocarboné ramifié est un groupement représenté par -C(Ra)(Rb)(Rc), où Ra, Rb et Rc représentent indépendamment un atome d'hydrogène, un groupement alkyle, un groupement cycloalkyle ou un groupement aryle, avec la condition que l'un au moins parmi Ra, Rb et Rc représente un groupement alkyle comptant un atome de fluor comme substituant, un groupement cycloalkyle comptant un atome de fluor comme substituant ou un groupement aryle comptant un atome de fluor comme substituant.

6. Composé polymérisable selon la revendication 1,
dans lequel, dans la formule (1), R₃ représente un groupement hydrocarboné cyclique dans lequel une partie ou tous les atomes d'hydrogène liés à un ou des atomes de carbone constituants sont substitués par un atome de fluor, et
le groupement hydrocarboné cyclique est un groupement phényle comptant un atome de fluor comme substituant et qui peut en outre porter un substituant, un groupement cyclopentyle comptant un atome de fluor comme substituant et qui peut en outre porter un substituant ou un groupement cyclohexyle comptant un atome de fluor comme substituant et qui peut en outre porter un substituant.

7. Composé contenant une lactone représentée par la formule (4) ci-dessous 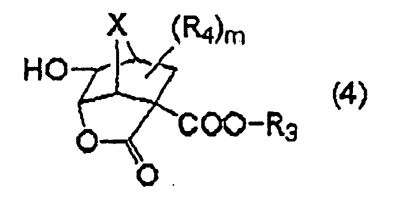 dans laquelle
R₃ représente un groupement hydrocarboné linéaire, ramifié ou cyclique, dans lequel un partie, ou tous les atomes d'hydrogène liés à un ou des atomes de carbone constituants sont substitués par un atome de fluor, et qui peut en outre porter un substituant,
R₄ représente un atome d'halogène, un groupement cyano, un groupement hydroxy, un groupement amide, un groupement alkyle qui peut porter un substituant, un groupement cycloalkyle qui peut porter un substituant, un groupement alkoxy qui peut porter un substituant, un groupement phényle qui peut porter un substituant, un groupement acyle qui peut porter un substituant, un groupement alkoxycarbonyle qui peut porter un substituant, ou un groupement représenté par R-C(=O)- ou R-C(=O)O-, où R représente un groupement alkyle qui peut porter un substituant, ou un groupement cycloalkyle qui peut porter un substituant et, lorsque plusieurs R₄ sont présents, les R₄ peuvent être identiques ou différents de tout autre R₄, et deux R₄ ou plus peuvent être liés l'un à l'autre pour former un cycle,
X représente un groupement alkylène qui peut porter un substituant, un atome d'oxygène ou un atome de soufre, et
m représente le nombre de substituants et est un entier de 0 à 7.

8. Procédé de fabrication d'un composé contenant une lactone représenté par la formule (4) ci-dessous, le procédé comprenant les étapes consistant à :
faire réagir un composé contenant de l'acide carboxylique représenté par la formule (5) ci-dessous avec un alcool représenté par la formule (6) ci-dessous : 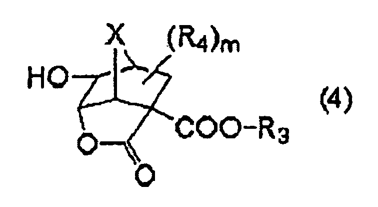 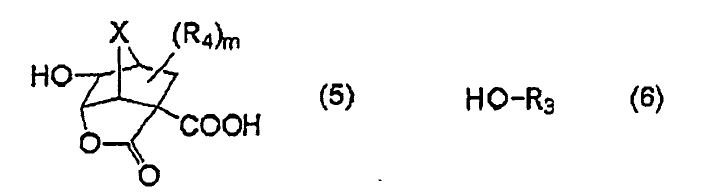 dans lesquelles
R₃ représente un groupement hydrocarboné linéaire, ramifié ou cyclique, dans lequel un partie, ou tous les atomes d'hydrogène liés à un ou des atomes de carbone constituants sont substitués par un atome de fluor, et qui peut en outre porter un substituant,
R₄ représente un atome d'halogène, un groupement cyano, un groupement hydroxy, un groupement amide, un groupement alkyle qui peut porter un substituant, un groupement cycloalkyle qui peut porter un substituant, un groupement alkoxy qui peut porter un substituant, un groupement phényle qui peut porter un substituant, un groupement acyle qui peut porter un substituant, un groupement alkoxycarbonyle qui peut porter un substituant, ou un groupement représenté par R-C(=O)- ou R-C(=O)O-, où R représente un groupement alkyle qui peut porter un substituant, ou un groupement cycloalkyle qui peut porter un substituant et, lorsque plusieurs R₄ sont présents, les R₄ peuvent être identiques ou différents de tout autre R₄, et deux R₄ ou plus peuvent être liés l'un à l'autre pour former un cycle,
X représente un groupement alkylène qui peut porter un substituant, un atome d'oxygène ou un atome de soufre, et
m représente le nombre de substituants et est un entier de 0 à 7.

9. Composé polymère obtenu en polymérisant le composé polymérisable de la revendication 1.
